# EUROPEAN PATENT SPECIFICATION

(11) **EP 0 833 902 B1**
(45) Date of publication and mention of the grant of the patent: **14.05.2003**
(21) Application number: 96920788.5
(22) Date of filing: 04.06.1996
(51) Int. Cl.: C12N 15/11, A61K 31/70, C12Q 1/70, C12Q 1/68, C07H 21/04

(54) **OLIGONUCLEOTIDES SPECIFIC FOR HEPATITIS C VIRUS**
SPEZIFISCHE OLIGONUKLEOTIDE FUER HEPATITIS C-VIRUS
OLIGONUCLEOTIDES SPECIFIQUES CONTRE LE VIRUS DE L'HEPATITE C

(30) Priority: 06.06.1995 US 471968
(43) Date of publication of application: 08.04.1998
(60) Divisional application: 03005364.9
(73) Proprietor: HYBRIDON, INC., Cambridge, Massachusetts 02139 (US)
(72) Inventor: FRANK, Bruce, L., Marlborough, MA 01752 (US); GOODCHILD, John, Westborough, MA 01581 (US); HAMLIN, Henry, A., Jr., Holland, MA 01521 (US); KILKUSKIE, Robert, E., Shrewsbury, MA 01545 (US); ROBERTS, Noel, A., Harpenden Herts. AL5 4TP (GB); ROBERTS, Peter, C., Holliston, MA 01746 (US); WALTHER, Debra, M., Newton, PA 18940 (US); WOLFE, Jia, L., Somerville, MA 02143 (US)
(74) Representative: Vossius, Volker, Dr.
(86) International application number: EP9602427
(87) International publication number: WO96039500

(56) References cited:
- WO-A-94/05813
- WO-A-94/08002
- WO-A-95/30746
- HEPATOLOGY 22 (3). 1995. 707-717. ISSN: 0270-9139, XP000603288 ALT M ET AL: "Specific Inhibition of Hepatitis C Viral Gene Expression by Antisense Phosphorothioate Oligodeoxynucleotides."
- THE JOURNAL OF BIOLOGICAL CHEMISTRY, vol. 269, no. 19, 13 May 1994, pages 14205-14210, XP000604710 T. WAKITA ET AL.: "Specific inhibition of Hepatitis C Virus expression by antisense oligonucleotides." cited in the application
- 46TH ANNUAL MEETING AND POSTGRADUATE COURSE OF THE AMERICAN ASSOCIATION FOR THE STUDY OF LIVER DISEASES, CHICAGO, ILLINOIS, USA, NOVEMBER 3-7, 1995. HEPATOLOGY 22 (4 PART 2). 1995. 330A. ISSN: 0270-9139, XP000603289 MORADPOUR D ET AL: "Specific inhibition of hepatitis C virus gene expression by antisense oligonucleotides in a tightly regulated cell culture system."
- BIOCHEMICAL AND BIOPHYSICAL RESEARCH COMMUNICATIONS 212 (3). 1995. 906-911. ISSN: 0006-291X, XP000604711 MIZUTANI T ET AL: "Inhibition of hepatitis C virus replication by antisense oligonucleotide in culture cells."
- NINTH INTERNATIONAL CONFERENCE ON ANTIVIRAL RESEARCH, URABANDAI, JAPAN, MAY 19-24, 1996. ANTIVIRAL RESEARCH 30 (1). 1996. A23. ISSN: 0166-3542, XP000603345 ANDERSON K P ET AL: "Antisense oligonucleotide -mediated inhibition of hepatitis C virus gene expression in transformed hepatocytes."

## Description

This invention relates to hepatitis C virus. More particularly, this invention relates to oligonucleotides complementary to particular regions of hepatitis C virus nucleic acid and to methods of inhibiting the expression and replication of hepatitis C virus nucleic acid and protein using these oligonucleotides.

Hepatitis C virus (HCV) is an enveloped, positive sense, single-stranded RNA virus which infects hepatocytes. HCV is the major cause of non-A, non-B, acute and chronic hepatitis (Weiner et al. (1990) Lancet 335:1-3), and has been associated with hepatocellular carcinoma (see, Dienstag et al. in Harrison's Principles of Internal Medicine, 13th Ed. (Isselbacher et al., eds.) McGraw-Hill, Inc. NY (1994) pp. 1458-1483).

The genome of HCV is a positive sense, single-stranded linear RNA of approximately 9,500 bases. The organization of this genome is similar to pestiviruses and flaviviruses, with structural proteins at the 5' end and non-structural proteins at the 3' end (reviewed by Houghton et al. (1991) Hepatol. 14:381-388). The viral RNA encodes a single polyprotein which is processed by viral and cellular proteases. HCV also contains short 5' and 3' untranslated regions (UTR). The 5' UTR is the most highly conserved region of the virus (Bukh et al. (1992) Proc. Natl. Acad. Sci. (USA) 89:4942-4946). This region has been shown to facilitate internal ribosomal entry, so that translation does not occur by ribosomal scanning from the 5' RNA cap. Instead, ribosomes bind to internal secondary structures formed by the 5' UTR (Wang et al. (1994) J. Virol. 68:7301-7307). In addition, separate experiments have shown that HCV 5' UTR sequences can control translation of downstream sequences (Yoo et al. (1992) Virol. 191:889-899). Recently, HCV was shown to replicate in cell culture (Yoo et al. (1995) J. Virol. 69:32-38).

HCV can be transmitted by transfusion and other percutaneous routes, such as self-injection with intravenous drugs. In addition, this virus can be transmitted by occupational exposure to blood, and the likelihood of infection is increased in hemodialysis units (Dienstag et al. in Harrison's Principles of Internal Medicine (13th Ed.) (Isselbacher et al., eds.) McGraw-Hill, Inc., NY (1994) pp. 1458-14843). The risk of HCV infection is also increased in organ transplant recipients and in patients with AIDS; in all immunosuppressed groups, levels of anti-HCV antibodies may be undetectable, and a diagnosis may require testing for HCV RNA. Chronic hepatitis C occurs in as many as 20 percent of renal transplant recipients. Five to 10 years after transplantation, complications of chronic liver disease account for increased morbidity and mortality (Dienstag et al., (ibid.).

Because there is no therapy for acute viral hepatitis, and because antiviral therapy for chronic viral hepatitis is effective in only a proportion of patients, emphasis has been placed on prevention through immunization (Dienstag et al., ibid.). However, for transfusion-associated hepatitis C, the effectiveness of immunoglobulin prophylaxis has not been demonstrated consistently and is not usually recommended.

Thus, there is a need for a treatment for HCV-induced hepatitis, and for methods of controlling HCV RNA and protein expression.

New chemotherapeutic agents have been developed which are capable of modulating cellular and foreign gene expression (see, Zamecnik et al. (1978) Proc. Natl. Acad. Sci. (USA) 75:280-284). These agents, called antisense oligonucleotides, bind to target single-stranded nucleic acid molecules according to the Watson-Crick rule or to double stranded nucleic acids by the Hoogsteen rule of base pairing, and in doing so, disrupt the function of the target by one of several mechanisms: by preventing the binding of factors required for normal transcription, splicing, or translation; by triggering the enzymatic destruction of mRNA by RNase H, or by destroying the target via reactive groups attached directly to the antisense oligonucleotide.

Improved oligonucleotides have more recently been developed that have greater efficacy in inhibiting such viruses, pathogens and selective gene expression. Some of these oligonucleotides having modifications in their internucleotide linkages have been shown to be more effective than their unmodified counterparts. For example, Agrawal et al. (Proc. Natl. Acad. Sci. (USA) (1988) 85:7079-7083) teaches that oligonucleotide phosphorothioates and certain oligonucleotide phosphoramidates are more effective at inhibiting HIV-1 than conventional phosphodiester-linked oligodeoxynucleotides. Agrawal et al. (Proc. Natl. Acad. Sci. (USA) (1989) 86:7790-7794) discloses the advantage of oligonucleotide phosphorothioates in inhibiting HIV-1 in early and chronically infected cells.

In addition, chimeric oligonucleotides having more than one type of internucleotide linkage within the oligonucleotide have been developed. Pederson et al. (U.S. Patent Nos. 5,149,797 and 5,220,007) discloses chimeric oligonucleotides having an oligonucleotide phosphodiester or oligonucleotide phosphorothioate core sequence flanked by nucleotide methylphosphonates or phosphoramidates. Agrawal et al. (WO 94/02498) discloses hybrid oligonucleotides having regions of deoxyribonucleotides and 2'-O-methylribonucleotides.

Antisense oligonucleotides have been designed that are complementary to portions of the HCV genome. For example, oligonucleotides specific for various regions of the HCV genome have been developed (see, e.g., CA 2,104,649, WO 94/05813, WO 94/08002 and Wakita et al. (1994) J. Biol. Chem. 269:14205-14210). Unfortunately, no demonstration has been made in any reasonably predictive system that any of these oligonucleotides are capable of inhibiting the replication and expression of hepatitis C Virus.

A need still remains for the development of oligonucleotides that are capable of inhibiting the replication and expression of hepatitis C virus whose uses are accompanied by a successful prognosis, and low or no cellular toxicity.

### BRIEF DESCRIPTION OF THE DRAWINGS

The objects of the present invention and the various features thereof may be more fully understood from the following description, when read together with the accompanying drawings in which:
FIG. 1 is a schematic representation of the HCV target mRNA sequence,
FIG. 2A is a diagrammatic representation of the proposed secondary structure of the HCV target mRNA sequence and one representative non-contiguous oligonucleotide of the invention;
FIG. 2B is a diagrammatic representation of the proposed secondary structure of the HCV target mRNA sequence and another representative non-contiguous oligonucleotide of the invention;
FIG. 3 is a schematic representation of the RNase H cleavage assay;
FIG. 4A is a graphic representation of HCV RNase H cleavage of Region B of HCV mRNA;
FIG. 4B is a graphic representation of HCV RNase H cleavage of Region A of HCV mRNA;
FIG. 4C is a graphic representation of HCV RNase H cleavage of Region C of HCV mRNA;
FIG. 5 is a graphic representation of RNase H cleavage of HCV mRNA stimulated by non-contiguous oligonucleotides, where (-□-) refers to results from an oligonucleotide where site 2 is on the '3' end of site 1, and (--◇--) refers to results from an oligonucleotide where site 2 is on the 5' and of site 1; X axis shows the location of 5' base of site 2 in relation to the start codon;
FIG. 6 is a graphic representation showing the effect of changing the anchor chemistry of a non-contiguous oligonucleotide of the invention on RNase H cleavage activity;
FIG. 7 is a graphic representation of RNase H cleavage of HCV mRNA in the presence of non-contiguous PS oligonucleotides competing with different concentrations of a specific non-contiguous 2' OMe oligonucleotide complementary to site 1;
FIG. 8 is a schematic representation of the HCV constructs used in various assays;
FIG. 9 is a graphic representation showing inhibition of HCVLUC in HepG2 HCVLUC cells where "-" is hcv1, SEQ ID NO:28, and "-x-" is a random 20mer (r20), at varying µM concentrations of oligonucleotide;
FIG. 10 is a graphic representation showing the inhibitory effect of different oligonucleotides of the invention (at 0.2 µM) on luciferase expression, wherein numbers within bars are the position of the 3' end of the oligonucleotide relative to the translation start site;
FIG. 11A is a phosphorimage of a ribonuclease protection assay gel showing the effect of oligonucleotides of the invention or a random 20mer on the amount of HCV-specific RNA using probe 1;
FIG. 11B is a phosphorimage of a ribonuclease protection assay gel showing the effect of oligonucleotides of the invention and a random 20mer on the amount of HCV-specific RNA using probe 2; and
FIG. 11C is a schematic representation of probes 1 and 2 used in the protection assays shown in FIGS. 11A and 11B and described in Table 4.

Antisense oligonucleotide technology provides a novel approach to the inhibition of HCV expression, and hence, to the treatment or prevention of chronic and acute hepatitis and of hepatocellular carcinoma (see generally, Agrawal (1992) Trends Biotech. 10:152; and Crooke (Proc. Am. Ass. Cancer Res. Ann. Meeting (1995) 36:655). By binding to the complementary nucleic acid sequence, antisense oligonucleotides are able to inhibit splicing and translation of RNA, and replication of genomic RNA. In this way, antisense oligonucleotides are able to inhibit protein expression.

The present invention provides oligonucleotides useful for inhibiting the replication of HCV or the expression of HCV genomic or messenger RNA or protein in a cell, and for treating HCV infection.

It has been discovered that specific oligonucleotides complementary to particular portions of the HCV genomic or messenger RNA can inhibit HCV replication or expression. This discovery has been exploited to provide synthetic oligonucleotides complementary to non-contiguous regions of the 5' untranslated region and/or to the 5' terminal end of the RNA encoding the HCV C protein.

For purposes of the invention, the term "oligonucleotide sequence that is complementary to genomic or mRNA" is intended to mean an oligonucleotide that binds to the nucleic acid sequence under physiological conditions, e.g., by Watson-Crick base pairing (interaction between oligonucleotide and single-stranded nucleic acid) or by Hoogsteen base pairing (interaction between oligonucleotide and double-stranded nucleic acid) or by any other means including in the case of a oligonucleotide binding to RNA, causing pseudoknot formation. Binding by Watson-Crick or Hoogsteen base pairing under physiological conditions is measured as a practical matter by observing interference with the function of the nucleic acid sequence.

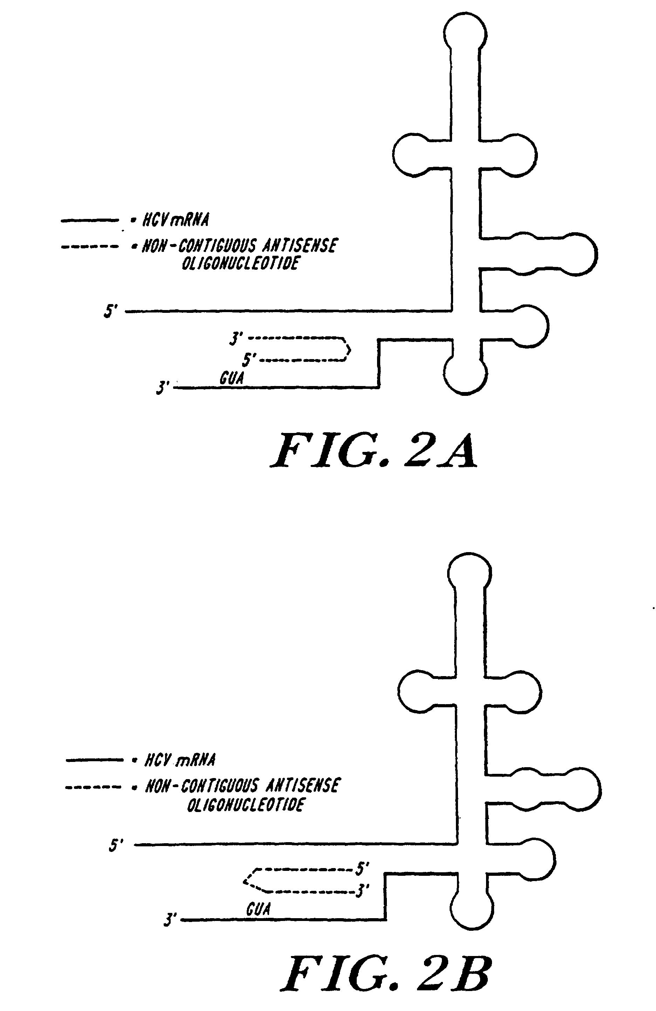

The present invention provides a synthetic oligonucleotide complementary to a sequence consisting of two non-contiguous regions of an HCV messenger or genomic RNA. Non-contiguous oligonucleotides are targeted to at least two regions of the HCV genomic RNA or mRNA which are not contiguous in a linear sense but, which may be next to each other in three dimensional space due to the secondary structure or conformation of the target molecule (FIGS. 2A and 2B). In preferred embodiments, one or both portions of the "non-contiguous" oligonucleotide is complementary to the 5' untranslated region. One portion of some non-contiguous oligonucleotides includes the same 12 bases (bases 100-111) designated the "anchor" region. The other portion of such non-contiguous oligonucleotides is variable, containing 6 to 12 bases within, e.g., bases 315-340 of HCV nucleic acid. In one embodiment, one portion which is complementary to the 5' untranslated region comprises the sequence GGGGUCCUGGAG (SEQ ID NO:47), and the other portion is complementary to a 5' region of the RNA encoding the HCV C protein. Other non-contiguous oligonucleotides of the invention may be targeted to other non-contiguous regions of HCV nucleic acid. For example, in another embodiment, the portion which is complementary to the 5' untranslated region and which functions as an anchor comprises the sequence CAACACUACUCG (bases 243-254). In preferred embodiments, the non-contiguous oligonucleotide has about 18 to about 24 nucleotides in length.

In a particular embodiment, the non-contiguous oligonucleotide which is complementary to two non-contiguous regions comprises one of the sequences as set forth in the Sequence Listing as SEQ ID NO:38, 39, 40, 41, 42, 43, 44, 45, 46, 48, 49, 50, 51, 52, 53, 54, 55, 56, 57, 58, 59, 60, 61, 62, 63, 64, 65, 66, and 67, or as set forth in Table 1C as SEQ ID NO: 134, 135, 136, 137, 138, 139, 140, 141, 142, 143, 144, 145, 146, 147.

In another embodiment of non-contiguous oligonucleotides of the present invention, an oligonucleotide may bind to three non-continuous regions. These oligonucleotides are called tripartite non-contiguous oligonucleotides (see for example, Table 1D). The tripartite oligonucleotides are developed as described herein for non-contiguous oligonucleotides using non-continuous oligonucleotides (as described herein) as a 2' OMe RNA anchor with a short semi-randomized DNA sequence attached. Where this short DNA sequence can bind is detected by cleavage with RNAase H as described herein, and the specific tripartite oligonucleotide of the invention may be designed. In particular, the invention provides corresponding oligonucleotides as set forth in Table 1D under SEQ ID NOS: 148, 149, 150, 151, 152, 153, 154, 155, 156, 157, 158.

In some embodiments, the oligonucleotides of the invention are modified. In one embodiment, these modifications include at least one internucleotide linkage selected from the group consisting of alkylphosphonate, phosphorothioate, phosphorpdithioate, alkylphosphonothioate, phosphoramidate, carbamate, carbonate, phosphate triester, acetamidate, or carboxymethyl ester including combinations of such linkages, as in a chimeric oligonucleotide. In one preferred embodiment, an oligonucleotide of the invention comprises at least one phosphorothioate internucleotide linkage. In another embodiment, the oligonucleotide comprises at least one or at least two inosine residues at any position in the oligonucleotide. In another embodiment, the oligonucleotide contains one or more 5-methyl-2'-deoxycytidine residues instead of the 2'deoxycytidine.

In another modification, the oligonucleotides of the invention may also include at least one deoxyribonucleotide, at least one ribonucleotide, or a combination thereof, as in a hybrid oligonucleotide. An oligonucleotide containing at least one 2'-O-methyl ribonucleotide is one embodiment of the invention. In another embodiment, the oligonucleotide consists of deoxyribonucleotides only. The oligonucleotides may be further modified as outlined below.

The oligonucleotides of the present invention are for use as therapeutically active compounds, especially for use in the control or prevention of hepatitis C virus infection. In other aspects, the invention, provides a pharmaceutical composition comprising at least one non-contiguous HCV-specific oligonucleotide of the invention as described above and below, and in some embodiments, this composition includes at least two different oligonucleotides (i.e., having a different nucleotide sequence, length, and/or modification(s)). The pharmaceutical composition of some embodiments is a physical mixture of at least two, and preferably, many oligonucleotides with the same or different sequences, modifications, and/or lengths. In some embodiments, this pharmaceutical formulation also includes a physiologically or pharmaceutically acceptable carrier.

In this aspect of the invention, a therapeutic amount of a pharmaceutical composition containing HCV-specific synthetic oligonucleotides is administered to the cell for inhibiting hepatitis C virus replication or of treating hepatitis C virus infection. The HCV-specific oligonucleotides are the non-contiguous oligonucleotides of the invention. In preferred embodiments, the use includes administering at least one non-contiguous oligonucleotide, or at least two non-contiguous oligonucleotides, having a sequence set forth in Table 2 or in the Sequence Listing as SEQ ID NO: 38, 39, 40, 41, 42, 43, 44, 45, 46, 48, 49, 50, 51, 52, 53, 54, 55, 56, 57, 58, 59, 60, 61, 62, 63, 64, 65, 66, and 67, or as set forth in Tables 1C-1E as SEQ ID NOS: 134-158 and 166-170, or a combination thereof. The oligonucleotides may also be used in modified form.

In all methods involving the administration of oligonucleotide(s) of the invention, at least one, and preferably two or more identical or different oligonucleotides may be administered simultaneously or sequentially as a single treatment episode in the form of separate pharmaceutical compositions.

In another aspect, the invention provides a method of detecting the presence of HCV in a sample, such as a solution or biological sample. In this method, the sample is contacted with a synthetic oligonucleotide of the invention. Hybridization of the oligonucleotide to the HCV nucleic acid is then detected if the HPV is present in the sample.

Another aspect of the invention are kits for detecting HCV in a sample. Such kits include at least one synthetic, non-contiguous oligonucleotide of the invention, which may have the same or different nucleotide sequence, length, and/or modification(s), and means for detecting the oligonucleotide hybridized with the nucleic acid.

As mentioned before, oligonucleotides of the invention are composed of deoxyribonucleotides, ribonucleotides, 2-O-methylribonucleotides, or any combination thereof, with the 5' end of one nucleotide and the 3' end of another nucleotide being covalently linked. These oligonucleotides are preferably 12 to 50 nucleotides long, with 20 to 30mers being the most common.

These oligonucleotides can be prepared by art recognized methods. For example, nucleotides can be covalently linked using art-recognized techniques such as phosphoramidite, H-phosphonate chemistry, or methylphosphonamidate chemistry (see, e.g., Goodchild (1990) Chem. Rev. 90:543-584; Uhlmann et al. (1990) Chem. Rev. 90:543-584; Caruthers et al. (1987) Meth. Enzymol. 154:287-313; U.S. Patent 5,149,798) which can be carried out manually or by an automated synthesizer and then processed (reviewed in Agrawal et al. (1992) Trends Biotechnol. 10:152-158).

The oligonucleotides of the invention may also be modified in a number of ways without compromising their ability to hybridize to HCV genomic or messenger RNA. For example, the oligonucleotides may contain other than phosphodiester internucleotide linkages between the 5' end of one nucleotide and the 3' end of another nucleotide in which other linkage, the 5' nucleotide phosphate has been replaced with any number of chemical groups, such as a phosphorothioate. Oligonucleotides with phosphorothioate linkages can be prepared using methods well known in the field such as phosphoramidite (see, e.g., Agrawal et al. (1988) Proc. Natl. Acad. Sci. (USA) 85:7079-7083) or H-phosphonate (see, e.g., Froehler (1986) Tetrahedron Lett. 27:5575-5578) chemistry. The synthetic methods described in Bergot et al. (J. Chromatog. (1992) 559:35-42) can also be used. Examples of other chemical groups, which can be used to form an internucleotide linkage, include alkylphosphonates, phosphorodithioates, alkylphosphonothioates, phosphoramidates, carbamates, acetamidate, carboxymethyl esters, carbonates, and phosphate triesters. As an example, for a combination of internucleotide linkages, US Patent No. 5,149,797 describes traditional chimeric oligonucleotides having a phosphorothioate core region interposed between methylphosphonate or phosphoramidate flanking regions. Other chimerics are "inverted" chimeric oligonucleotides comprising one or more nonionic oligonucleotide regions (e.g alkylphosphonate and/or phosphoramidate and/or phosphotriester internucleoside linkage) flanked by one or more regions of oligonucleotide phosphorothioates. Chimerics and inverted chimerics may be synthesized as discussed in the Examples for methyl phosphonate containing oligonucleotides. These "chimerics" and "inverted chimeric" oligonucleotides are a preferred embodiment for the modification of the oligonucleotides of the present invention.

Various oligonucleotides with modified internucleotide linkages can be prepared according to known methods (see, e.g., Goodchild (1990) Bioconjugate Chem. 2:165-187; Agrawal et al. (1988) Proc. Natl. Acad, Sci. (USA) 85:7079-7083; Uhlmann et al. (1990) Chem. Rev. 90:534-583; and Agrawal et al. (1992) Trends Biotechnol. 10:152-158).

Oligonucleotides which are self-stabilized are also considered to be modified oligonucleotides useful in the methods of the invention (Tang et al. (1993) Nucleic Acids Res 20; 2729-2735). These oligonucleotides comprise two regions: a target hybridizing region; and a self-complementary region having an oligonucleotide sequence complementary to a nucleic acid sequence that is within the self-stabilized oligonucleotide. These oligonucleotides form looped structures which are believed to stabilize the 3' end against exonuclease attack while still allowing hybridization to the target.

On the other hand, examples of modifications to sugars include modifications to the 2' position of the ribose moiety which include but are not limited to 2'-O-substituted with an -O- lower alkyl group containing 1-6 saturated or unsaturated carbon atoms, or with an -O-aryl, or allyl group having 2-6 carbon atoms wherein such -O-alkyl, aryl or allyl group may be unsubstituted or may be substituted (e.g., with halo, hydroxy, trifluoromethyl, cyano, nitro acyl acyloxy, alkoxy, carboxy, carbalkoxyl, or amino groups), or with an amino, or halo group. None of these substitutions are intended to exclude the native 2'-hydroxyl group in case of ribose or 2'-H- in the case of deoxyribose. PCT Publication No. WO 94/02498 discloses traditional hybrid oligonucleotides having regions of 2'-O-substituted ribonucleotides flanking a DNA core region.

Another form of a hybrid is an "inverted" hybrid oligonucleotide which includes an oligonucleotide comprising a 2'-O-substituted (or 2' OH, unsubstituted) RNA region which is interposed between two oligodeoxyribonucleotides regions, a structure that is inverted relative to the "traditional" hybrid oligonucleotides. Hybrid and inverted hybrid oligonucleotides may be synthesized as described in the Examples for oligonucleotides containing 2'-0-methyl RNA. The hybrid and inverted hybrid oligonucleotides of the invention are particularly preferred due to the enhanced stability and activity over time in the presence of serum. In another embodiment the hybrid or inverted hybrid may comprise at least one n-butyl phosphoramidate or methylphosphonate linkage.

Preferably, the ribonucleotide is a 2-0-methyl ribonucleotide. In another embodiment, the oligoriucleotide comprises at least one, preferably one to five 2-0-methyl ribonucleotides at the 3' end of the oligonucleotide. Moreover, the oligonucleotide may further comprise at least one, preferably one to five 2-0-methyl ribonucleotides at the 5'-end.

Other oligonucleotide structures of the invention include the so-called dumbell and nicked dumbell structures. Ashly and Kushlan (Biochem. (1991) 30:2927-2933) describe the synthesis of oligonucleotide dumbells including nicked dumbells. A dumbell is a double-helical stem closed off by two hairpin loops. The antisense activity of nicked dumbells (dumbell molecules with free ends) is discussed by Yamakawa et al. (Nucleosides and Nucleotides (1996) 15:519-529). These oligonucleotides structures are believed to have beneficial properties similar to those of the self-stabilized oligonucleotides described above.

In another aspect the present invention relates to non-contiguous multiplex oligonucleotides which are designed to target a polypurine or polypyrimidine sequence by a combination of duplex and triplex formation. In some cases, the multiplex oligonucleotide of the invention may be branched by adding linkers for supporting branched moieties as is known in the art. The multiplex oligonucleotides of the invention need not be continuous and may bind to two or more proximal sites as described herein for non-contiguous oligonucleotides.

Preferred non-contiguous multiplex oligonucleotides of the invention having SEQ ID NOS: 166-170 are shown in Table 1E. These oligonucleotides target the double strand RNA stem at -217 to -209 and the adjacent polypyrimidine sequence between -218 and -222. The hybridization of an antisense sequence to the single stranded polypyrimidine target creates a polypurine-polypyrimidine , duplex that can be targeted by a triplex motif to increase the oligonucleotide binding strength. These oligonucleotides therefore provide a portion of the triplex target by duplex formation with the RNA as well as the third strand of the triple helix. The multiplex oligonucleotides as designed contain an RNase H active portion for irreversible inactivation of the target RNA. The asymmetric branching amidite (Y) (Clone Tech. Palo Alto, California) is incorporated during solid phase synthesis and hydrolyzed with hydrazine monohydrate according to the manufacturer's instructions. The branching strand is added subsequently by the same solid phase approach.

Other modifications include those which are internal or are at the end(s) of the oligonucleotide molecule and include additions to the molecule of the internucleoside phosphate linkages, such as cholesteryl, cholesterol or diamine compounds with varying numbers of carbon residues between the two amino groups, and terminal ribose, deoxyribose and phosphate modifications which cleave, or crosslink to the opposite chains or to associated enzymes or other proteins which bind to the viral genome. Other examples of modified oligonucleotides include oligonucleotides with a modified base and/or sugar such as arabinose instead of ribose, or a 3', 5'-substituted oligonucleotide having a sugar which, at one or both its 3' and 5' positions is attached to a chemical group other than a hydroxyl or phosphate group (at its 3' or 5' position).

Additionally, oligonucleotides capped with ribose at the 3' end of the oligonucleotide may be subjected to NaIO₄ oxidation/reductive amination. Amination may include but is not limited to the following moieties, spermine, spermidine, Tris(2-aminoethyl) amine (TAEA), DOPE, long chain alkyl amines, crownethers, coenzyme A, NAD, sugars, peptides, dendrimers.

In another embodiment, at least one cytosine base may be modified by methylation as is known in the art, e.g., 5-methylated deoxycytosine (5-Me-dC) (see Table 1B). Such methylation may be desirable, for example, to reduce immune stimulation by the oligonucleotide if necessary.

Other modified oligonucleotides are capped with a nuclease resistance-conferring bulky substituent at their 3' and/or 5' end(s), or have a substitution in one or both nonbridging oxygens per nucleotide. Such modifications can be at some or all of the internucleoside linkages, as well as at either or both ends of the oligonucleotide and/or in the interior of the molecule (reviewed in Agrawal et al. (1992) Trends Biotechnol. 10:152-158), some non-limiting examples of capped species include 3' O-methyl, 5' O-methyl, 2' O-methyl, and any combination thereof, as shown in Table 1B

Examples of some preferred non-contiguous oligonucleotides of the invention are listed below in Tables 1C-1E. The contiguous oligonucleotides shown in tables 1A and 1B are only for illustrative purposes. In these Tables the internucleotide linkage is PS unless otherwise mentioned.

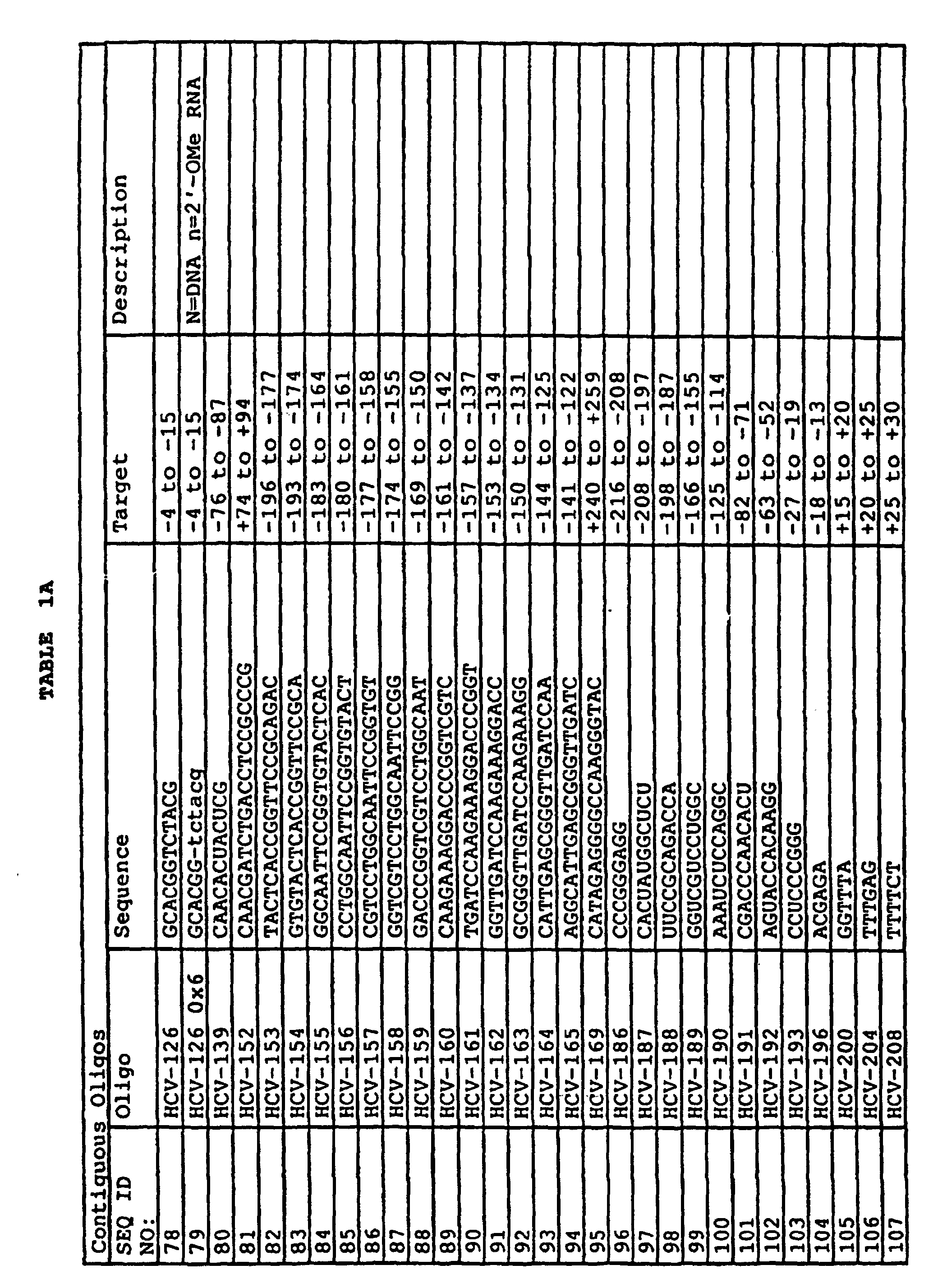

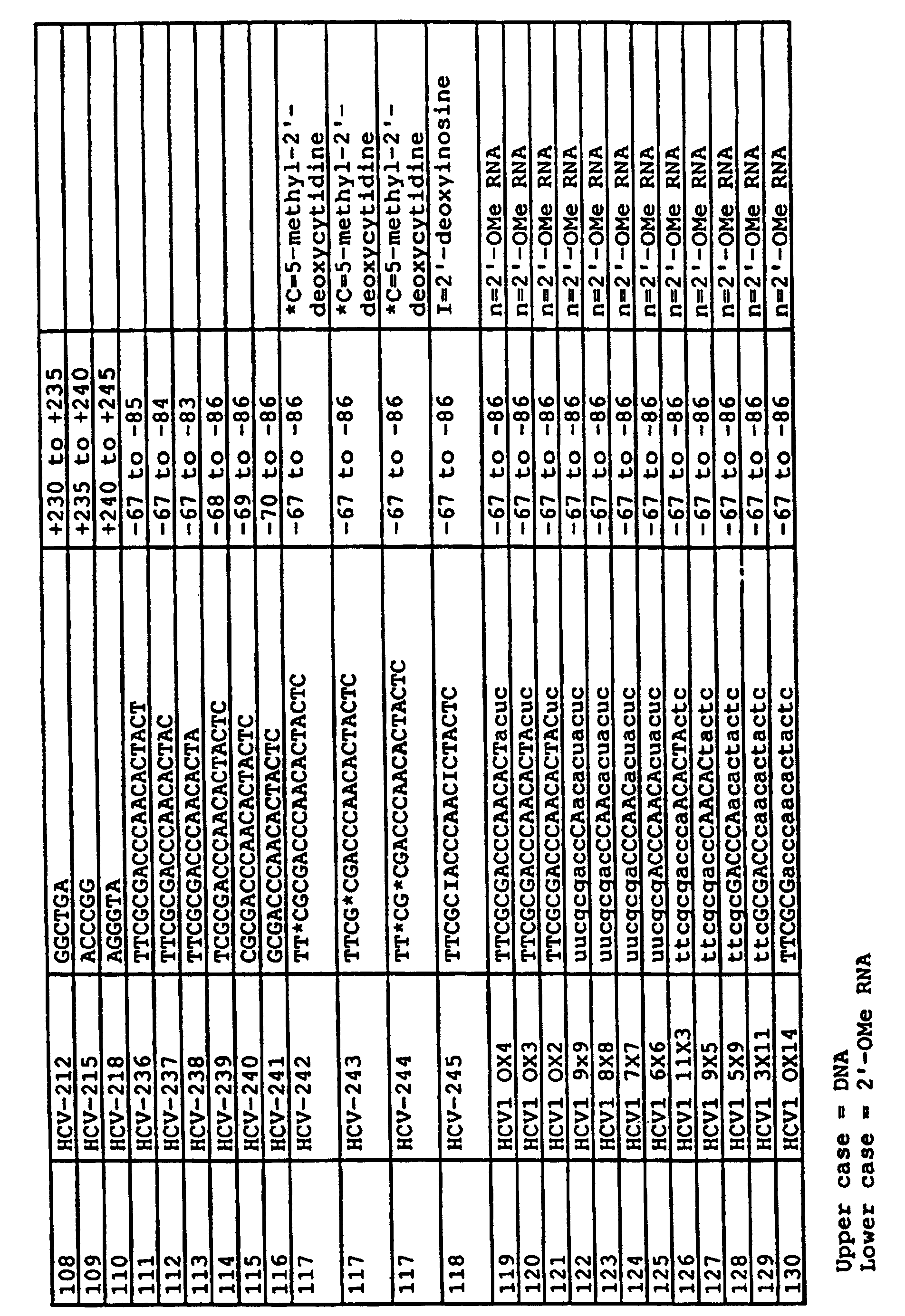

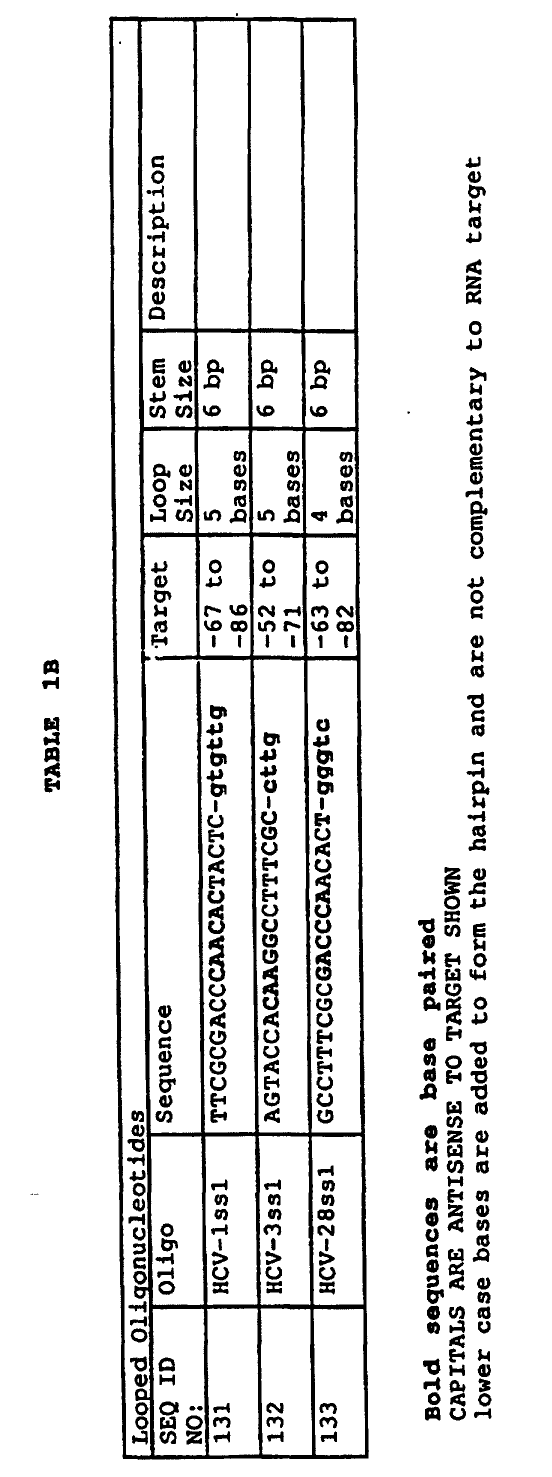

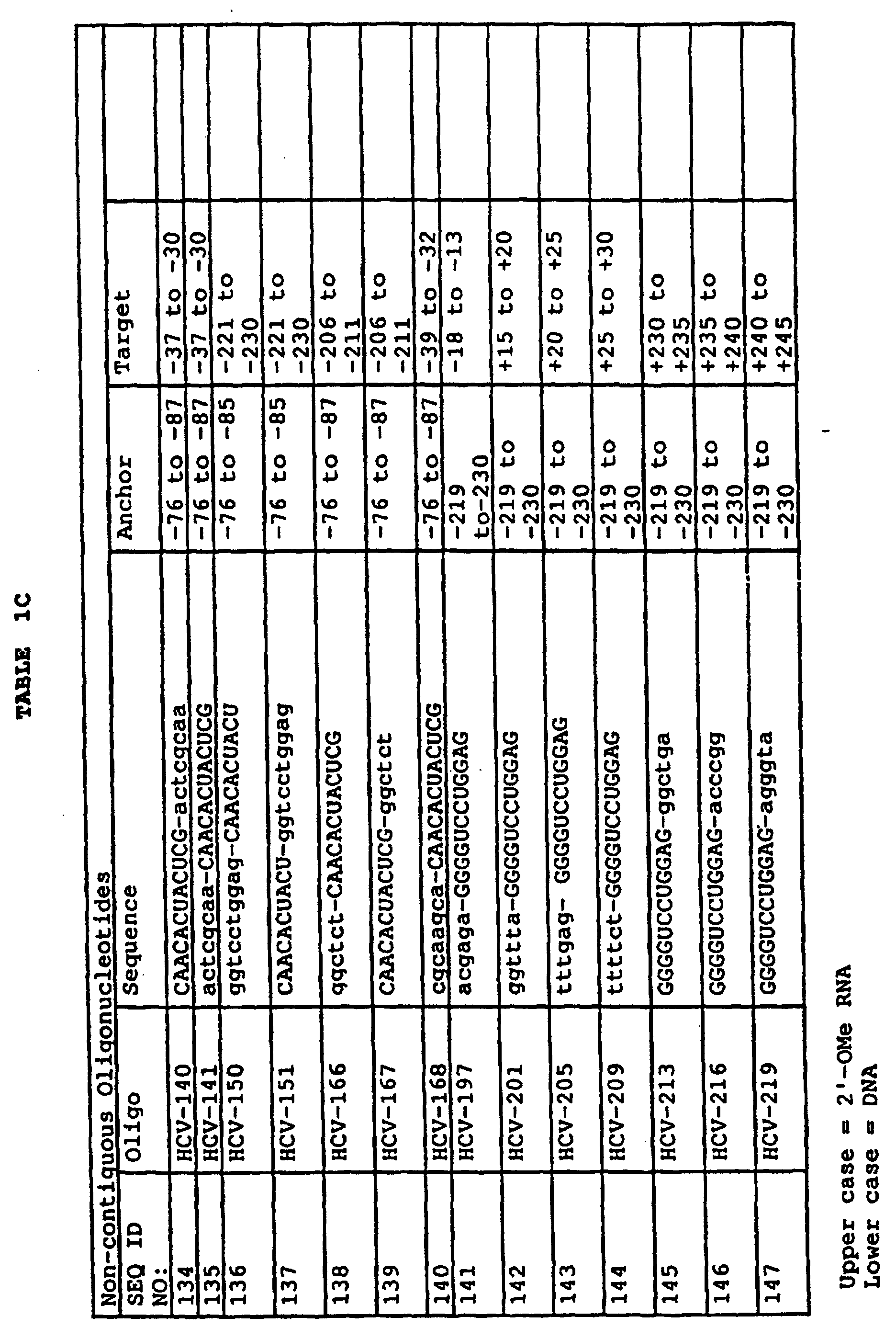

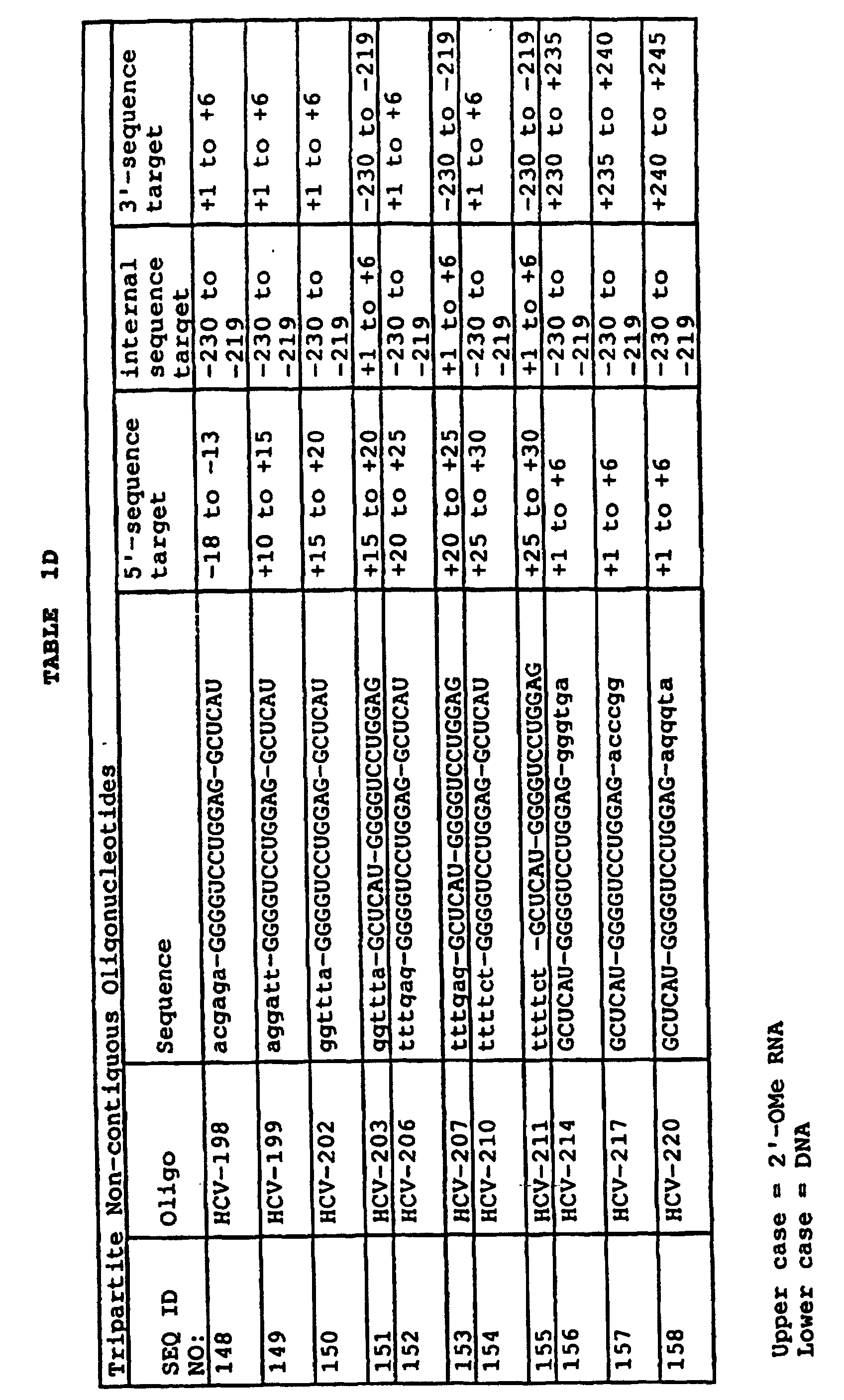

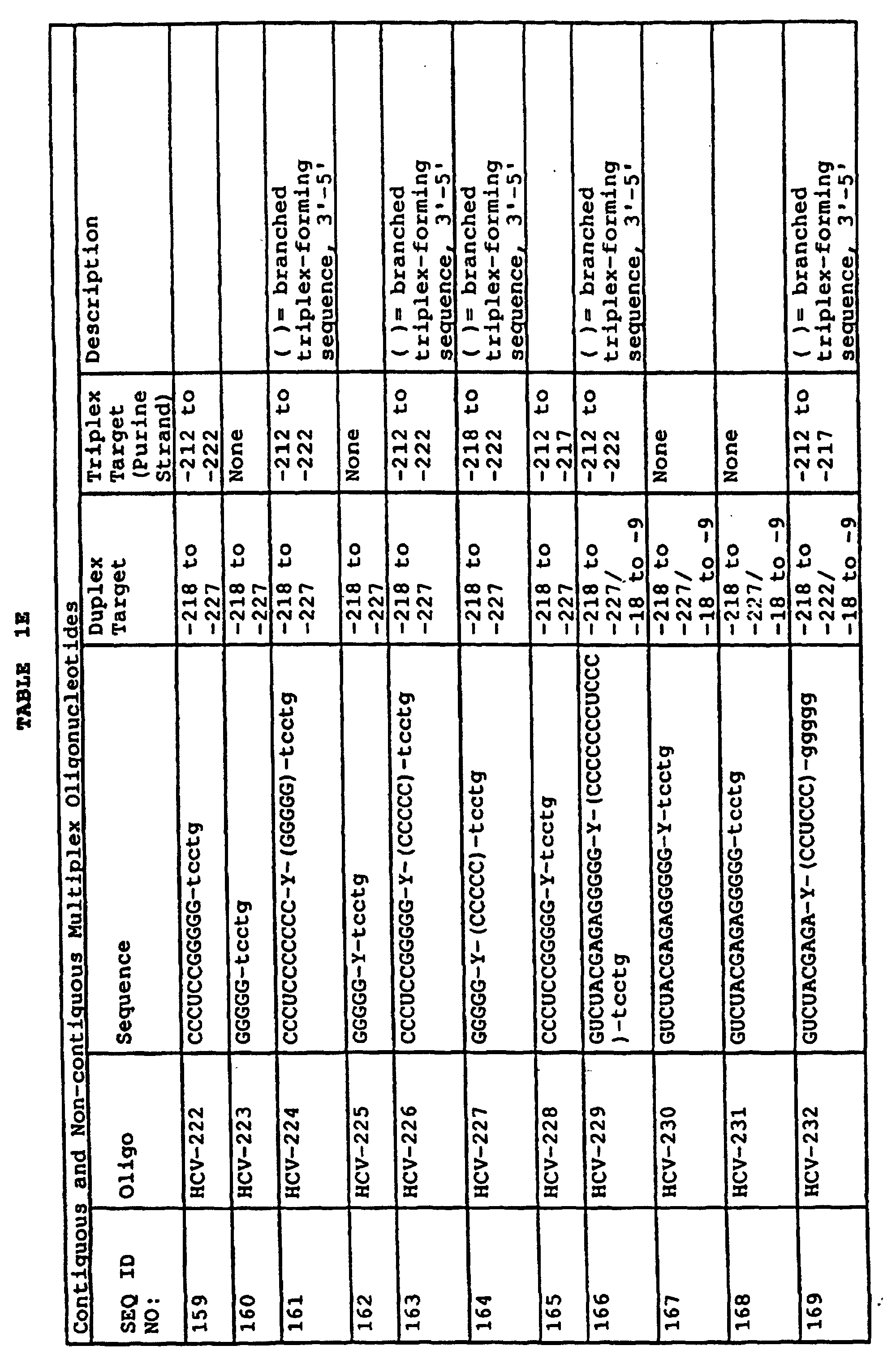

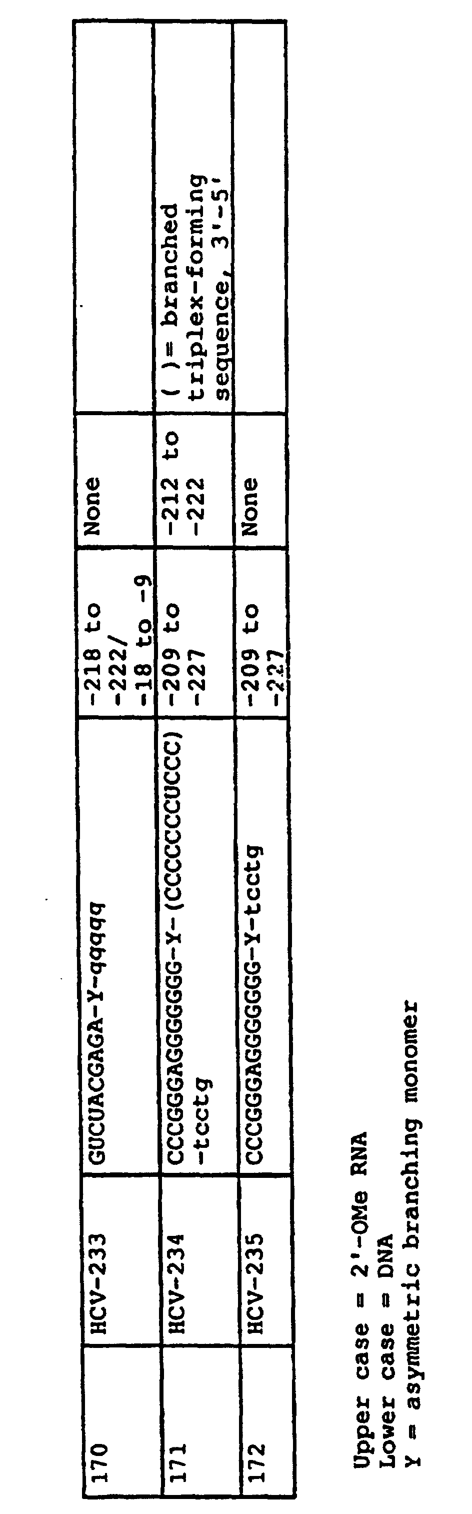

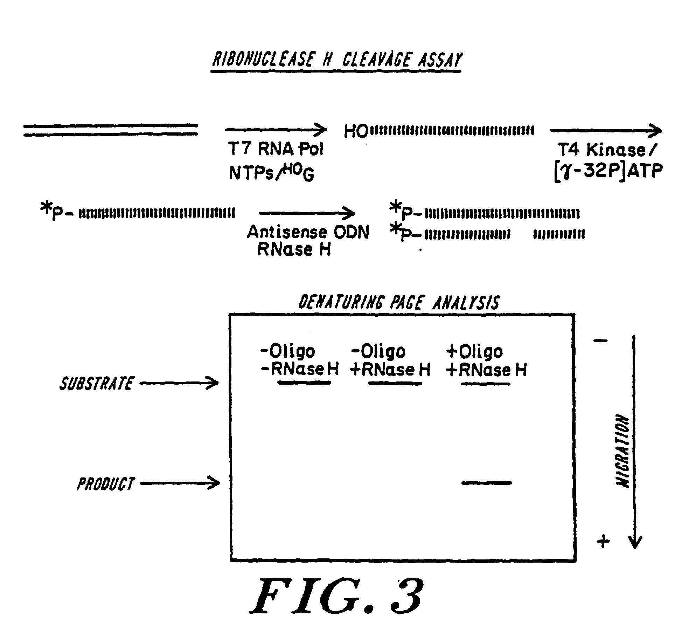

To determine whether an oligonucleotide of the invention is capable of successfully binding to its target, several assays can be performed. One assay is an RNase H assay (Frank et al. (1993) Proc. Int. Conf. Nucleic Acid Med. Applns. 1:4.14(abstract)) which is useful when a region of at least four contiguous nucleotides of the oligonucleotide is DNA and the target is RNA. Binding of the DNA portion of the oligonucleotide (ODN) to the RNA target is identified by cleavage at that site by RNase H, as shown schematically in FIG. 3.

Using this assay, three regions of HCV mRNA were investigated as RNase H sensitive areas, and were shown to be susceptible to hybridization by members of a degenerate 20mer library, Regions A, B, and C. The assay was performed with several oligodeoxynucleotide phosphorothioate 20mers targeted to these three regions and present at a concentration of 100 nM. The assays relating to contiguous oligonucleotides are only comparative examples. Contiguous oligonucleotides set forth in Table 1F are only for illustrative purposes.

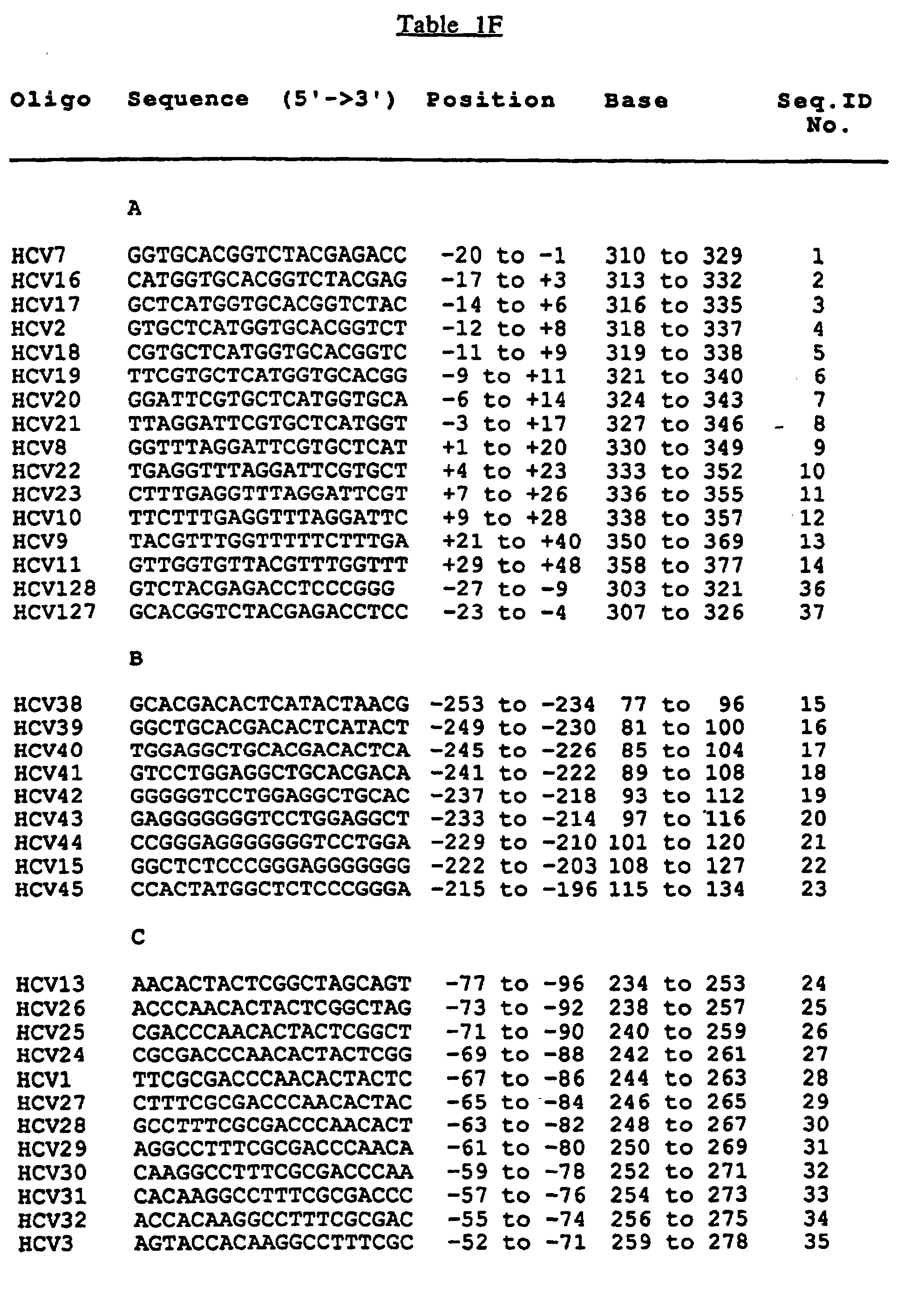

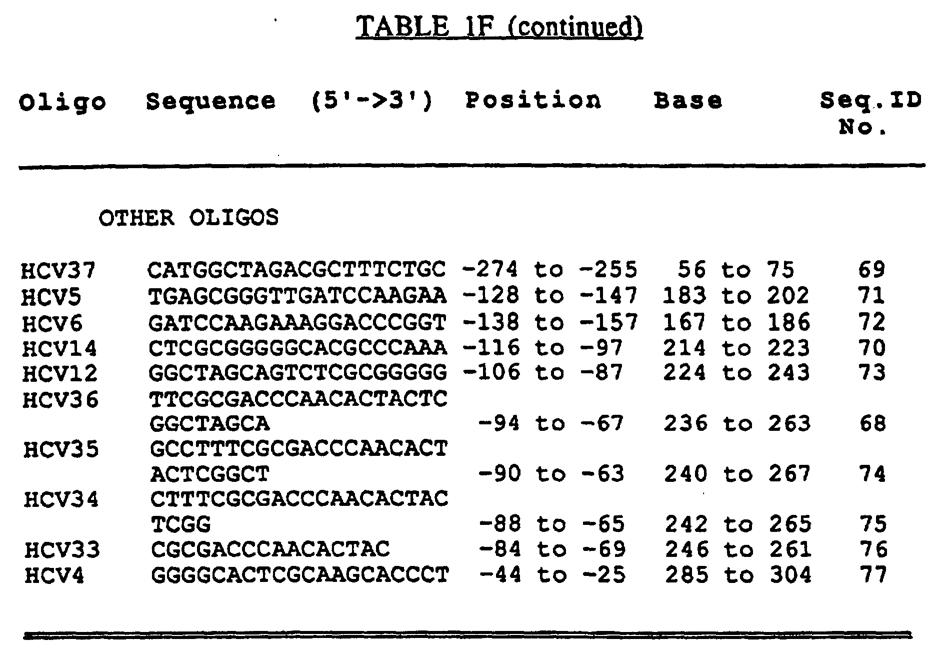

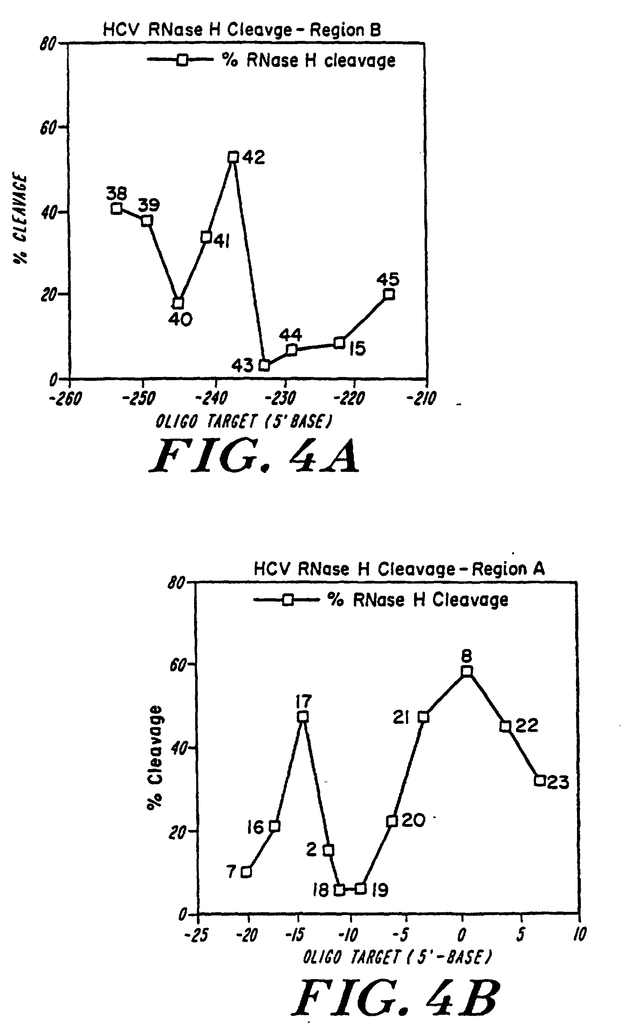

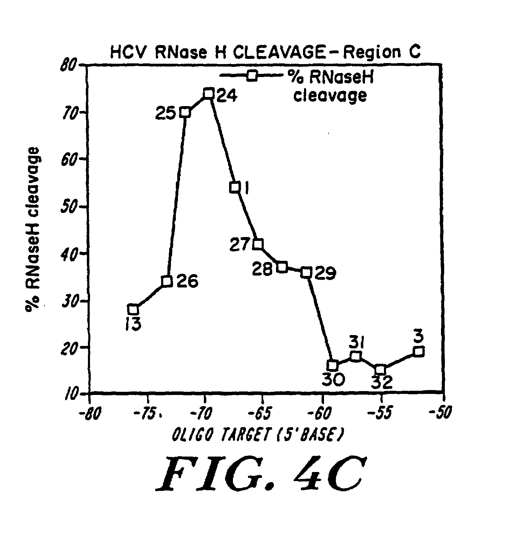

Region A (or site 2) (located around the start codon) shows two peaks of activity in the RNase H cleavage assay with oligonucleotides targeted to -12 to +8 and +1 to +20 (FIG. 4B). Region B (or site 1) (located upstream at approximately bases 210-260) shows a single peak of activity that corresponds to an oligonucleotide 20mer from -237 to -218 (FIG. 4A). Region C (located upstream at bases 50-80) shows one peak of activity in this assay, for oligonucleotides targeted to -69 to -88 (FIG. 4C).

When the secondary structure of the oligonucleotides was examined, it was noted that the valley of activity between the peaks in Region A corresponds to oligonucleotides with stably folded stem-loops (ΔG<-2 Kcal/mol). This suggests that secondary structure within the oligonucleotide can impede its ability to bind.

In order to determine whether the accessible sites found in the random library experiment could be used to reach other non-contiguous sites, a sequence in Region B was selected as the anchor for a semirandom oligonucleotide probe (SOP). The SOP has a defined 2'-OMe RNA "anchor" sequence complementary to bases -219 to -230 in Region B and a six base random DNA "tail" on either its 5' or 3' end. The 2'-OMe RNA portion cannot activate RNase H cleavage and a six base random DNA library without the anchor does not activate RNase H cleavage of the transcript under these conditions. RNase H cleavage only occurs by the anchor-facilitated binding of the six-base DNA tail to the target. These semirandom oligonucleotides efficiently activate RNase H cleavage at several sites, including near the anchor, near the start codon (Region A) and within the coding region of the mRNA.

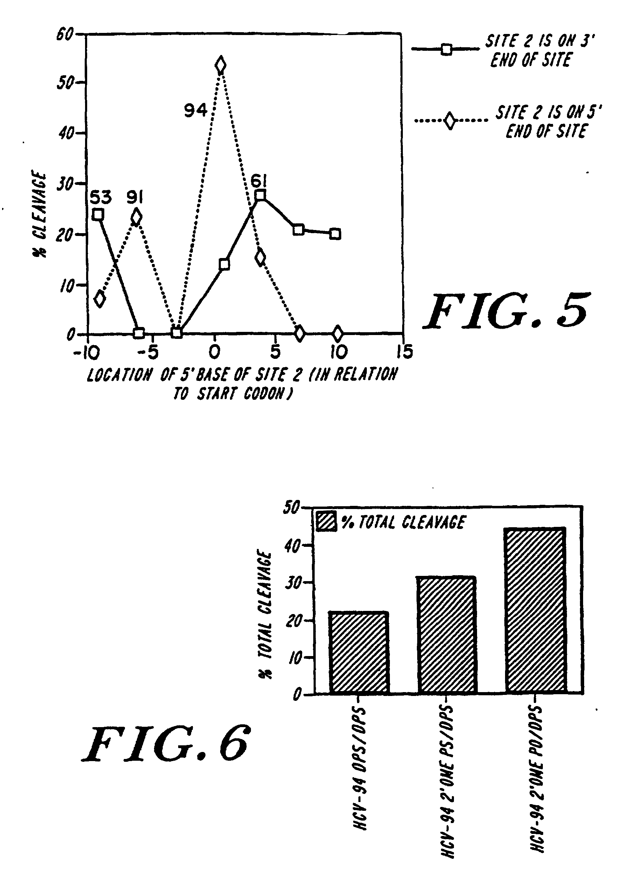

Using Region B as an anchor, Region A was targeted with non-contiguous oligonucleotide probes (NOPs). A series of NOPs were prepared that were able to bridge between Regions A and B. Maintaining the 2'-OMe anchor of the semirandomers (-219 to -230) allowed the sequence of the six base tail and the site of attachment to the anchor to be varied to find the best bridging sequence. The results of this experiment suggests that attaching the tail to different ends of the anchor gives a different optimal sequence, as shown by the different peaks of activity with RNase H. (FIG. 5).

The chemistry of the anchor of one NOP was modified to examine its effect on the binding strength of the tail. As shown in FIG. 6, modification of the 2'-OMe phosphodiester (PO) anchor to 2'-OMe phosphorothioate (PS) and DNA PS effected the cleavage efficiency of the tail. Cleavage paralleled the expected binding strength of the anchor, 2'-OMe PO > 2'-OMe PS > DNA PS.

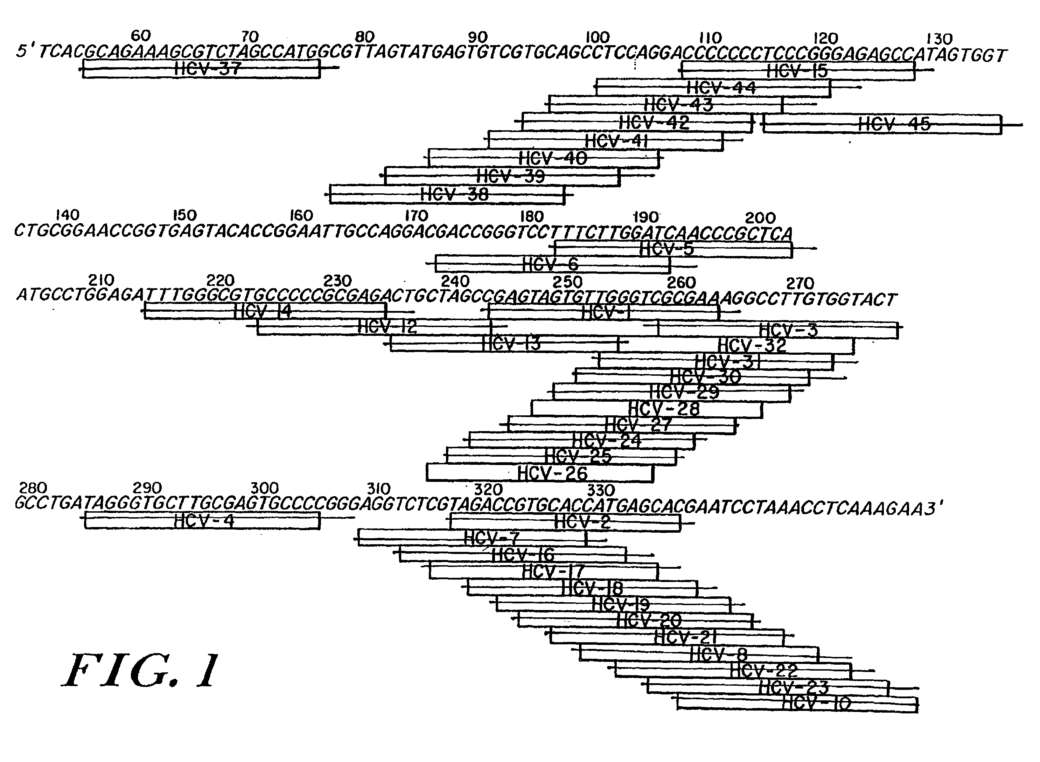

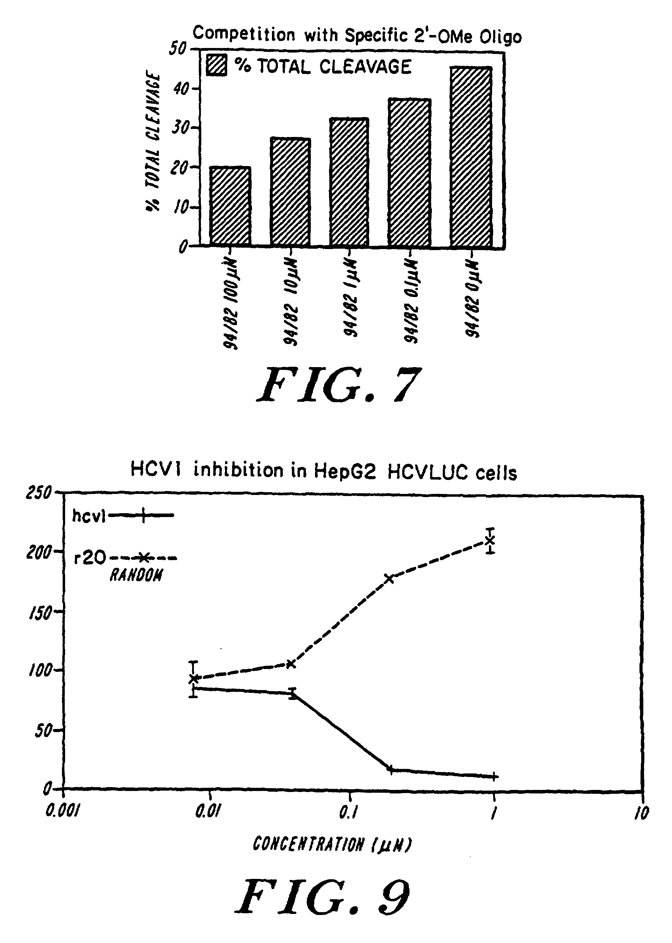

In order to establish the necessity of anchor binding for hybridization of the tail, a competition experiment was performed. In this experiment the binding of the anchor had to compete with increasingly higher concentrations of 2'-OMe PO 12mer of the same sequence. If binding of the anchor and tail are cooperative, the cleavage by the tail should decrease as the anchor is displaced by competitor (HCV82 (SEQ ID NO:47)). As seen in FIG. 7, cleavage of RNA decreases as the concentration of competitor increases. Surprisingly, a 1000-fold excess of competitor over NOP decreases cleavage only from 46% to 20%. This suggests that the 6 base tail imparts significant binding strength to the anchor so as to compete for Region B. More than 40 contiguous oligonucleotide sequences were evaluated as antisense inhibitors of HCV 5, UTR-dependent protein expression (FIG. 1). Some of these oligonucleotides had different chemical backbone modifications. These oligonucleotides were evaluated in three cellular assay systems: (1) inhibition of HCV luciferase (HCVLUC) fusion protein expression in stably transfected cells; (2) inhibition of HCV RNA expression in stably transfected cells; and (3) inhibition of HCV protein expression in Semliki Forest virus/HCV recombinant virus infected cells. They were also evaluated in RNase H cleavage.

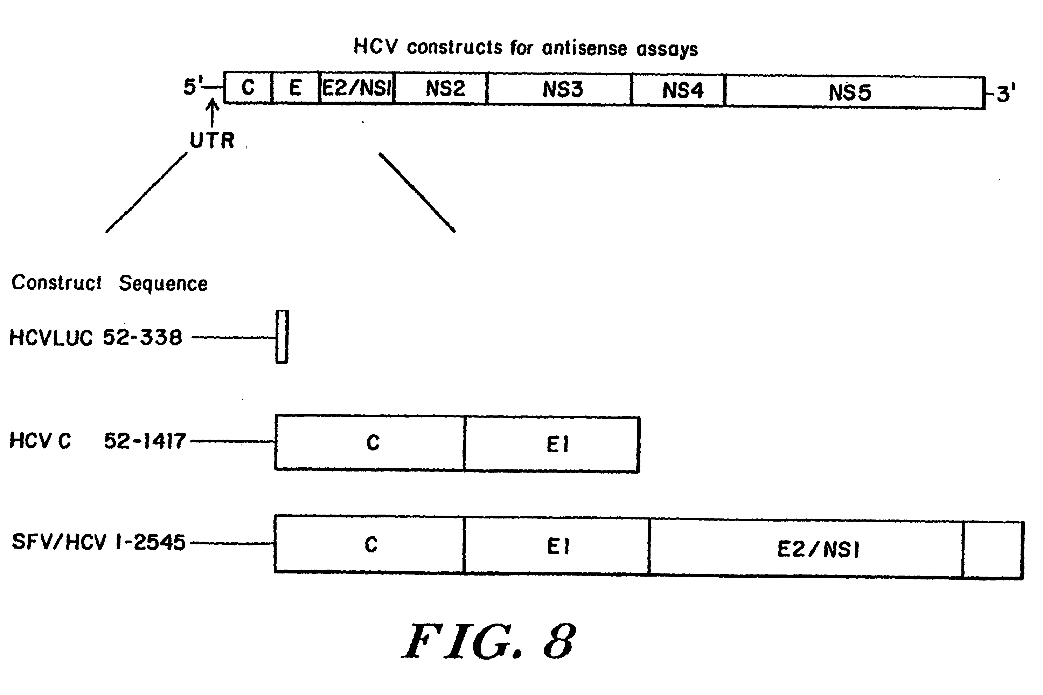

In the luciferase assay, the 5' UTR region of HCV containing the ATG start site was cloned 5' to the open reading frame of firefly luciferase (FIG. 8). Transcription of this HCV-luciferase gene fusion is stimulated in mammalian cells by a strong constitutive CMV promoter. Translation of the fusion gene is initiated at the HCV ATG which replaced the native luciferase ATG, and produces a protein which contains the first three amino acids of the viral protein and 648 amino acids of luciferase. Expression of this enzyme in mammalian cells, including the native host cells for HCV infection, can be quantified easily in a luminometer by addition of luciferin substrate and ATP cofactor to the lysed cells. Antisense oligonucleotides, when added to mammalian cells expressing this fusion construct, will reduce luciferase activity if these compounds target sequences within the 5' UTR of HCV and/or luciferase.

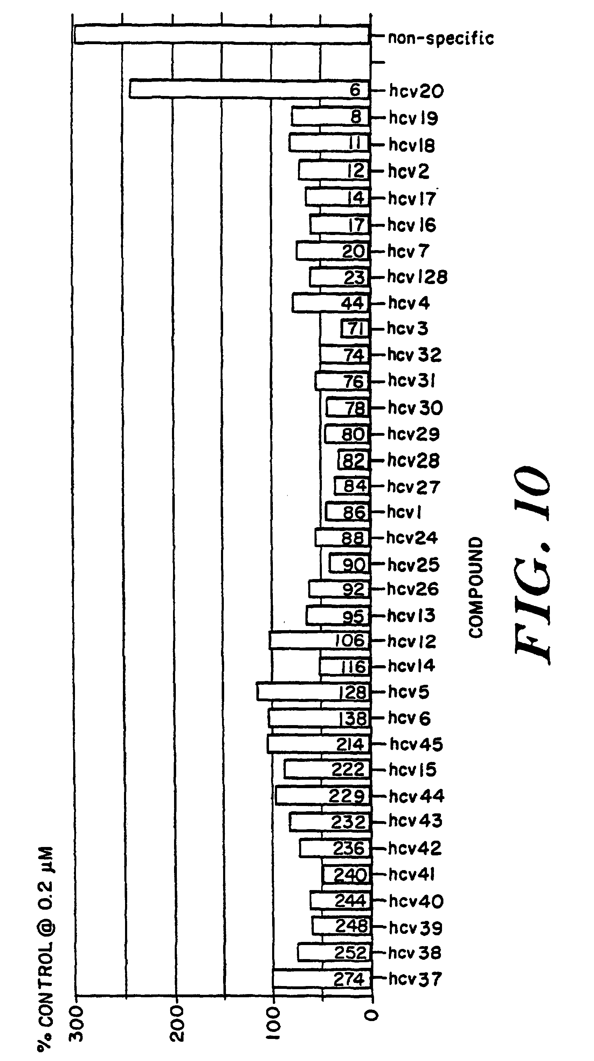

Both contiguous and non-contiguous oligonucleotides showed sequence specific inhibition of luciferase expression in HCVLUC cells. FIG. 9 shows a dose response for inhibition by oligonucleotide HCV1 (SEQ ID NO:28). This oligonucleotide is antisense to HCV sequences 244 to 263 (-86 to -67 relative to the start of translation for HCV) (see FIG. 1 and Table 1). Under these assay conditions, HCV1 inhibited luciferase by more than 50% at 1 and 0.2 µM relative to cells treated without oligonucleotide. No inhibition was observed at 0.04 and 0.008 µM. In the same experiment, a random 20mer (synthesized by including all four nucleotide phosphoramidites in every step of synthesis) did not inhibit but instead enhanced luciferase at 1 µM and 0.2 µM (FIG. 9).

These results suggest that inhibition was sequence specific. Additional oligonucleotides were evaluated to extend this observation. Sense (5' -> 3'), scrambled (3' -> 5'), and mismatched oligonucleotides did not inhibit HCVLUC under conditions that HCV1 inhibited by greater than or equal to 50%. These oligonucleotides all enhanced luciferase expression at concentrations where HCV1 inhibited luciferase. These results confirm that the inhibition was highly sequence specific.

A series of oligonucleotides targeted at different sequences in the 5' UTR were evaluated in this assay system (FIG. 1). Dose response curves (1 µM to 0.008 µM) were developed for all oligonucleotide sequences. In all oligonucleotides tested, 0.2 µM was the lowest concentration which showed significant luciferase inhibition. A summary of the inhibition at 0.2 µM is shown in FIG. 10. Not all oligonucleotides targeted against HCV 5' UTR sequences inhibited luciferase expression. More active oligonucleotides (for example, HCV1 and HCV3) had percent control values less than or equal to 50 percent in these experiments. Several oligonucleotides (for example, HCV37 (SEQ ID NO:69) and HCV14 (SEQ ID NO:70) had percent control values greater than 100 percent. The most active oligonucleotides were HCV1, HCV3, and HCV28. All are targeted in the same region, HCV sequences 240 to 290. A second region, HCV sequences 80 to 140, also was complementary to oligonucleotides that inhibited luciferase.

All oligonucleotides evaluated in this assay were designed to bind to HCV sequences. Since HCVLUC created a fusion between HCV and luciferase sequences 9 bases into the coding sequence, oligonucleotides HCV8, HCV10, and HCV19-23 all had greater than 4 mismatches with the HCVLUC sequence. None of these oligonucleotides inhibited luciferase expression. These results also confirm that sequence specific interaction with the target was required for luciferase inhibition.

Non-contiguous oligonucleotides were also evaluated in this assay. Oligonucleotides HCV53 (SEQ ID NO:39), HCV112 (SEQ ID NO:64), and HCV125 (SEQ ID NO:66), were tested and found to inhibit HCVLUC by greater than or equal to 50% at 1 µM. In addition to the anchor region, HCV53 targeted bases 324 to 329; HCV112 targeted sequences 324 to 335. This region may be particularly important for inhibition in these non-contiguous oligonucleotides.

These and other representative non-contiguous oligonucleotides of the invention are listed below in Table 2.

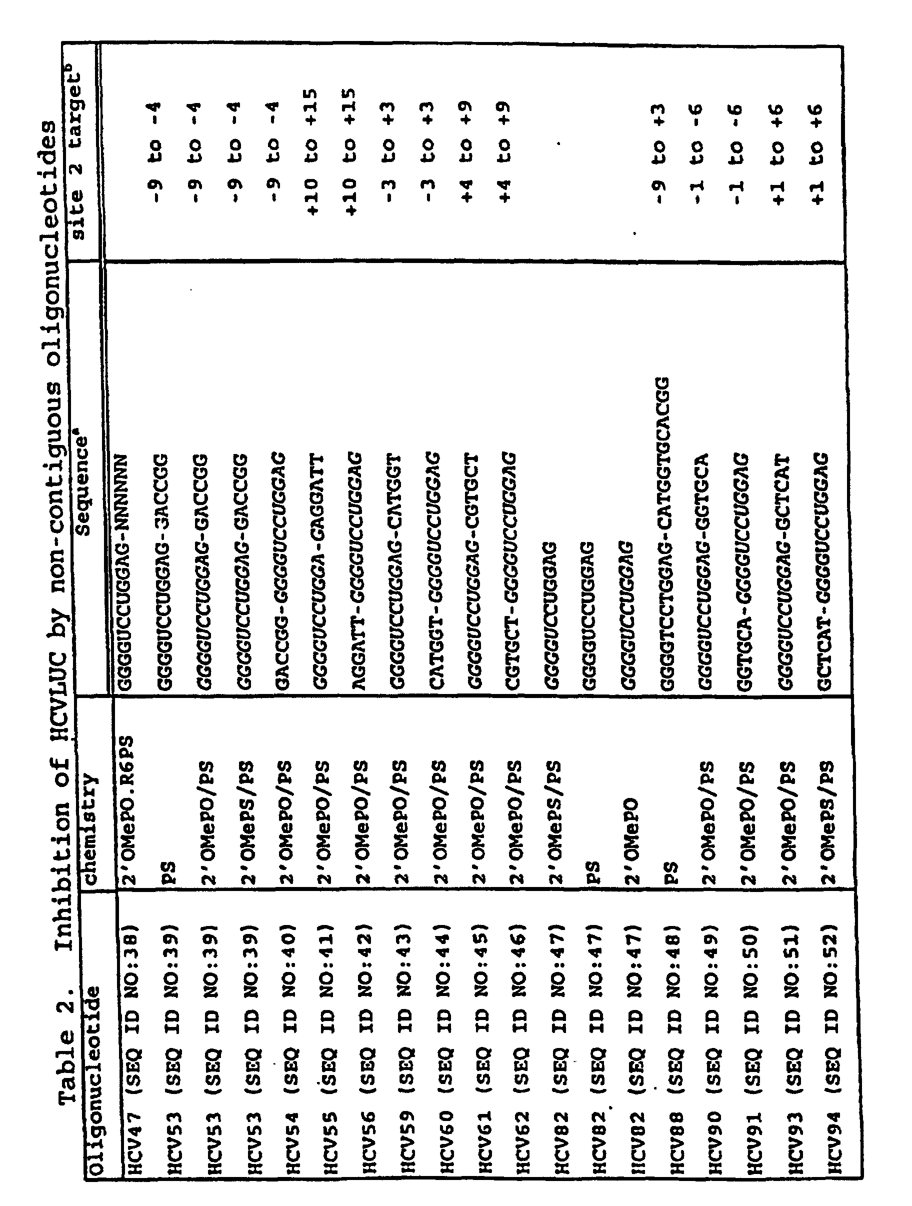

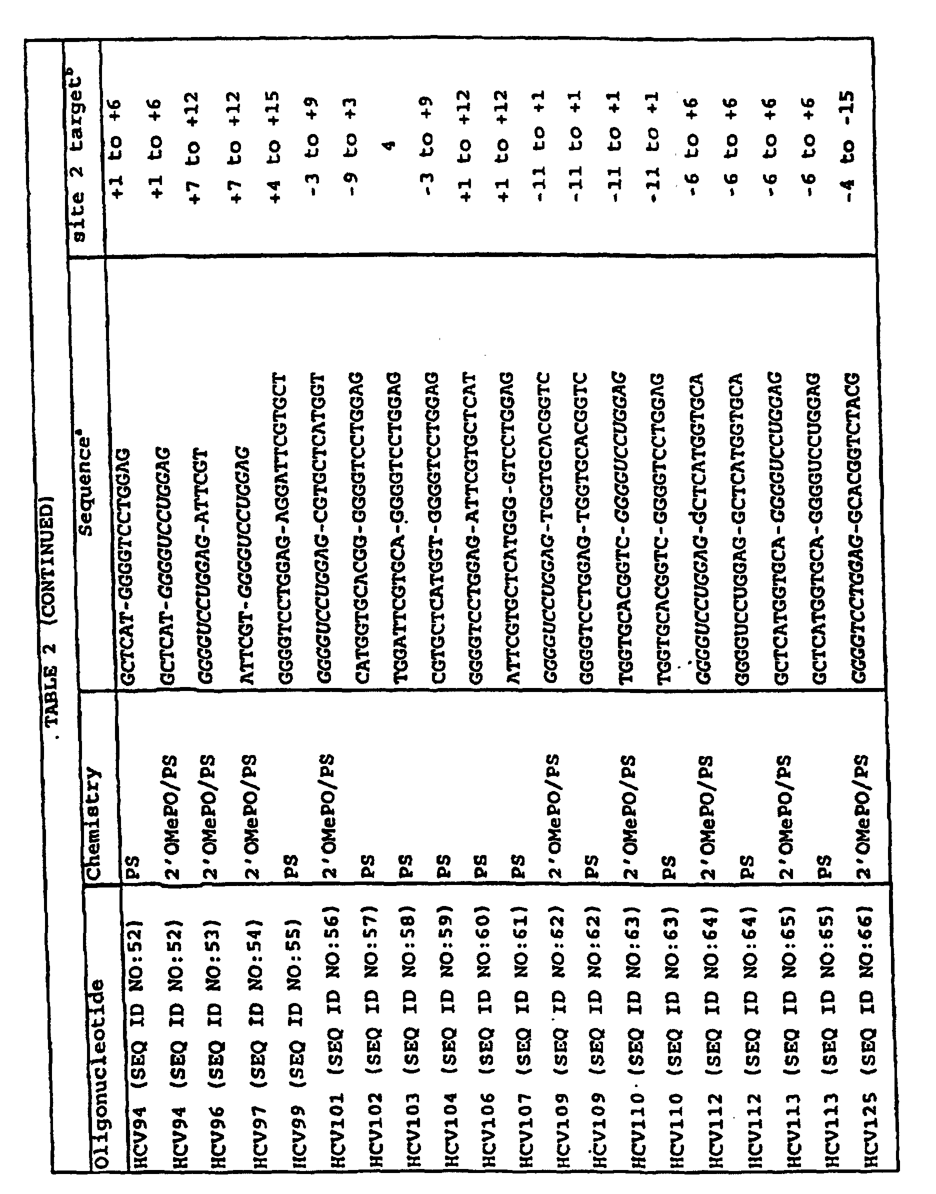

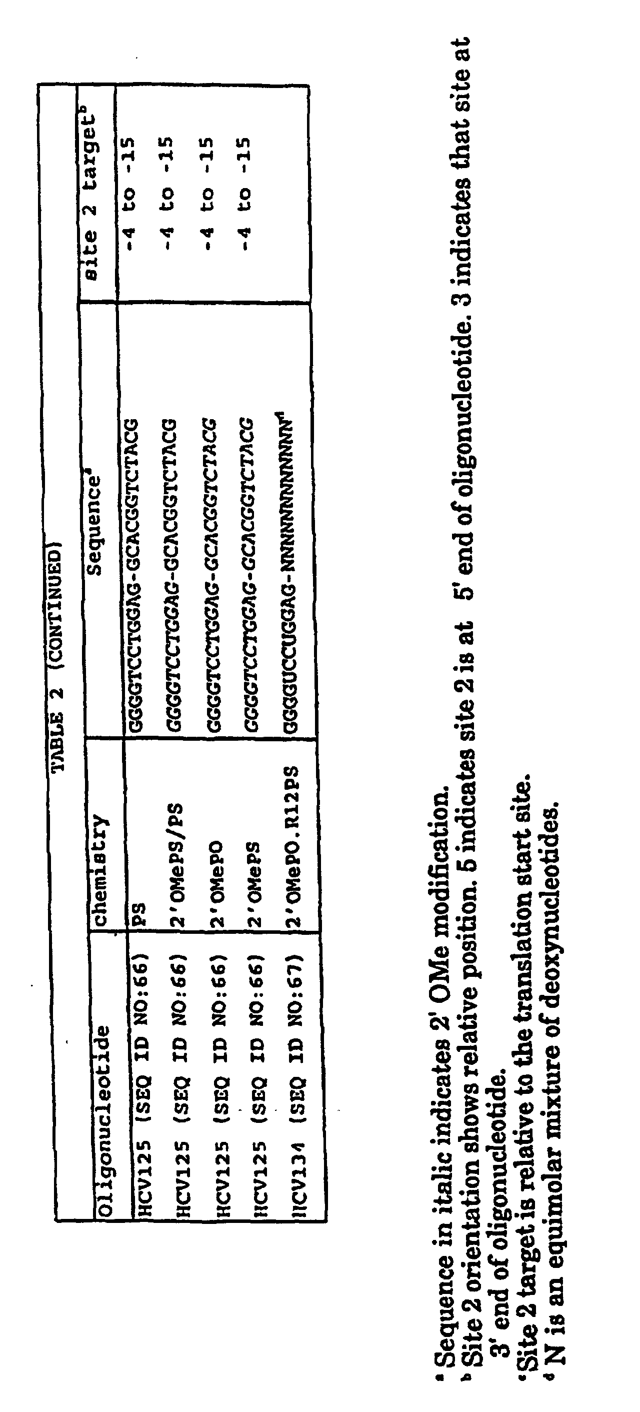

Oligonucleotides targeted at the HCV 5' untranslated region inhibited translation of a protein which was fused to the 5' untranslated region sequence. A longer HCV construct was also evaluated. This construct contained HCV sequences 52-1417, which encoded the C and E1 protein of HCV. The HCV construct was used to evaluate antisense oligonucleotide interaction with a larger HCV RNA. It was believed that this RNA secondary structure might resemble the HCV viral RNA more closely than the HCVLUC RNA. RNA levels were measured after oligonucleotide treatment to directly evaluate the interaction of oligos with their target.

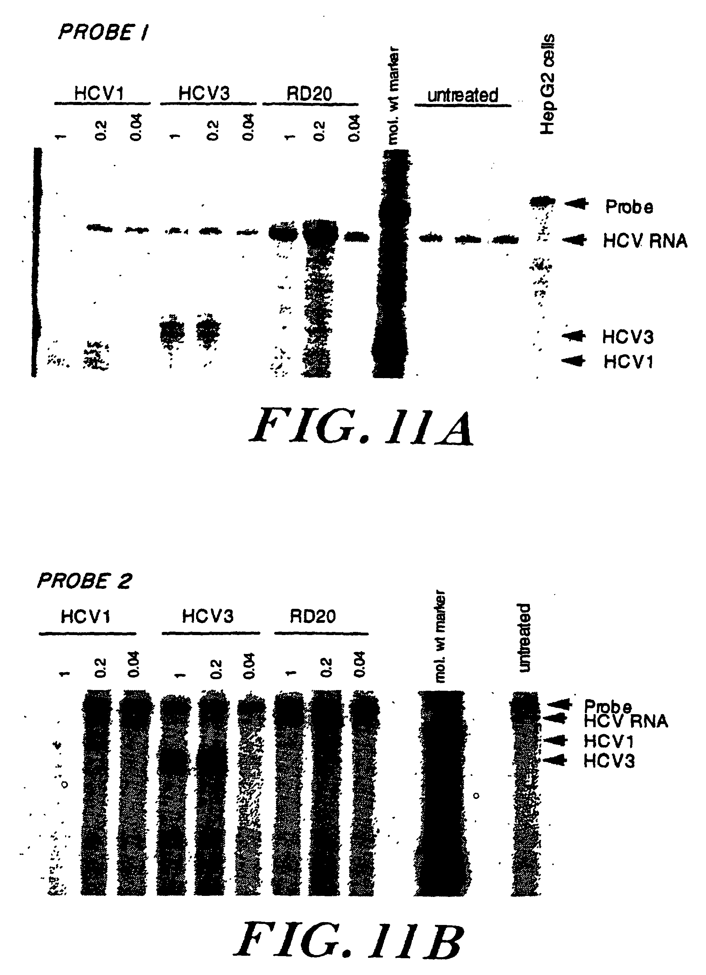

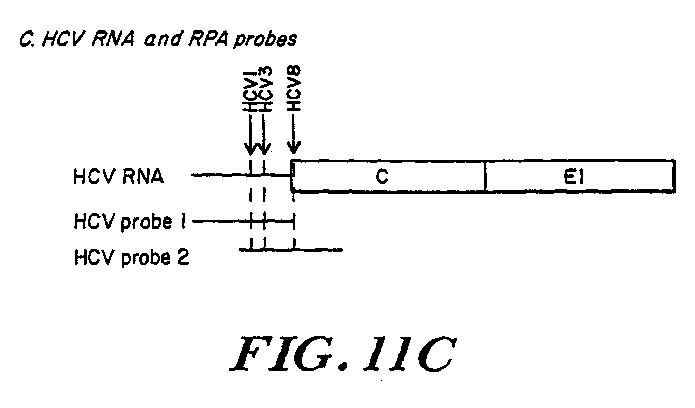

Treatment of HepG2 HCV (52-1417) cells with antisense oligonucleotide decreased the amount of HCV specific RNA, as shown in FIGS. 11A and 11B. HepG2 cells which were not transfected with the HCV construct do not produce a specific, HCV related band with probe 1 (FIG. 11A). Similar experiments were conducted to show the specificity of probe 2 (FIG. 11B). FIG. 11A and 11B show that HCV1 and HCV3 decreased HCV RNA in HCV (52-1417) cells. The amounts of full length HCV RNA were quantitated on the phosphorimager and compared to untreated cells (Table 3).

**TABLE 3**

| % untreated ^{a} | | | |
|---|---|---|---|
| Oligonucleotide | Concentration (µM) | Probe 1 | Probe 2 |
| HCV1 (SEQ ID NO:28) | 1.0 | 0 | 21 |
| | 0.2 | 47 | 69 |
| | 0.04 | 92 | 77 |
| HCV3 (SEQ ID NO:35) | 1.0 | 38 | 60 |
| | 0.2 | 54 | 72 |
| | 0.04 | 55 | 63 |
| R20 (random) | 1.0 | 316 | 254 |
| | 0.2 | 454 | 471 |
| | 0.04 | 126 | 125 |

| | | | |
|---|---|---|---|
| ^{a} Intensity of the HCV RNA band in each oligonucleotide treated sample was compared to the intensity of the untreated sample. | | | |

Full length RNA was decreased by greater than or equal to 80% in cells treated with 1 µM HCV1. HCV1 and HCV3 decreased RNA levels by greater than 40% at concentrations greater than or equal to 0.2 µM. Random oligonucleotide increased HCV RNA by greater than 3 fold at concentrations greater than or equal to 0.2 µM. These results are consistent with the sequence specific decrease and the nonspecific increase seen in luciferase in HepG2 HCVLUC cells (see above). In cells treated with HCV1 and HCV3 at greater than or equal to 0.2 µM, lower molecular weight bands were visible. These bands corresponded to the size of RNA which would result from RNase H cleavage of the HCV RNA/HCV1 duplex (see vertical dashed line in FIG. 11C). With probe 1, the 5' side of the apparent cleavage was visible, since the lower molecular weight band was 85-90 bases less than the full length RNA for HCV1 and 70-75 bases less than full length RNA for HCV3. HCV1 and HCV3 were targeted to HCV RNA sequences 75-94 and 60-80 bases from the 3' end of the RNA/probe hybrid. With probe 2, the 3' side of the cleavage was present; the lower molecular weight band was about 10 bases less than the full length RNA for HCV1 and 30-40 bases less than full length for HCV3. HCV1 and HCV3 were targeted to sequences 6-25 and 21-40 bases from the 5' end of the RNA/probe hybrid. Also, HCV1 and HCV3 are targeted to HCV RNA sequences 15 bases apart. The lower molecular weight bands detected on the gel were consistently about 15 bases apart.

The results from ribonuclease protection assays were consistent with specific oligonucleotide binding to target RNA. Neither probe by itself identified both cleavage products. The shorter fragments were not visible, probably because of their small size and non-specific background on the gel. Sequence specific degradation of HCV RNA confirmed the antisense activity of HCV1 and HCV3. The presence of cleavage products suggests that RNase H contributed to the activity of these phosphorothioate oligonucleotides in this assay system.

To confirm this observation, oligonucleotide specific RNA cleavage in cells was compared to in vitro cleavage of RNA/oligonucleotide hybrids by RNase H. HCV RNA was transcribed in vitro witl. T7 RNA polymerase and incubated with specific oligonucleotides and RNase H. RNA was then precipitated, and ribonuclease protection assays performed. Assays were performed as described above except that 0.1 ng in vitro transcribed RNA was used in the ribonuclease protection assay. Molecular weights of bands were determined by comparison to RNA standards.

As with oligonucleotide treated cells, specific lower molecular weight products were detected after in vitro RNase H cleavage of oligonucleotide/RNA hybrids. Molecular weights were consistent with predicted oligonucleotide binding sites and also with products detected in cells, as shown in Table 4.

**TABLE 4**

| Size comparison of in vitro and cellular RNA treated with oligonucleotides | | | |
|---|---|---|---|
| Unit | HCV1 (SEQ ID NO:28) | HCV3 (SEQ ID NO:35) | HCV8 (SEQ ID NO:10) |
| probe 1 | | | |
| predicted product^{a} | 75-94 | 60-79 | 8 |
| in vitro product^{b} | 93-97 | 71-80 | 7 |
| cellular product^{c} | 87-95 | 72-78 | 6 |

| probe 2 | | | |
|---|---|---|---|
| predicted product^{a} | 6-25 | 21-40 | 92-111 |
| in vitro product^{b} | 13-17 | 21-30 | 90-100 |
| cellular product^{c} | 10-30 | 30-40 | n.d. |

| | | | |
|---|---|---|---|
| ^{a} Predicted product is the molecular weight difference between the full length RNA and the RNA remaining after oligonucleotide binding and RNase cleavage. | | | |
| ^{b} In vitro product is the molecular weight difference between full length RNA and RNA detected after in vitro RNase H cleavage in the presence of oligonucleotide. | | | |
| ^{c} Cellular product is the molecular weight difference between full length RNA and RNA detected after treatment of target containing cells with oligonucleotide. | | | |

With probe 1 (FIG. 11C), HCV1 produced bands 90-95 bases less than full length RNA; HCV3 produced bands 70-80 bases less than full length RNA. With probe 2 (FIG. 11C), products were 13-17 bases less than full length for HCV1, 20-30 bases less than full length for HCV3. In summary, these results show that oligonucleotides inhibited RNA production by sequence-specific interaction with target RNA, and subsequent degradation by cellular RNase H.

SFV/HCV recombinant virus was prepared as a model system for measuring HCV protein production after virus infection. pSFV1/HCV (containing HCV sequence 1-2545) was prepared from a plasmid (Hoffman-Roche, Basel, Switzerland) and pSFV1 (Gibco/BRL, Gaithersburg, MD). RNA transcribed from pSFV1/HCV produces SFV replicase proteins which replicate the input RNA and produce multiple copies of subgenomic mRNA. The subgenomic RNA contains the 5' end of HCV RNA plus approximately 50 bases derived from the pSFV1 vector. This model has the advantages of cytoplasmic replication and a 5' end very similar to authentic HCV.

Recombinant SFV/HCV infected three cell types: HepG2; CHO; and BHK21. Infection was monitored by HCV C protein production. Cells were infected for 1 hour, inoculum was removed, and cells were cultured overnight. Cells were lysed and protein separated on a 13.3% polyacrylamide/SDS gel. Proteins were electroblotted onto nitrocellulose and detected by Western blot using rabbit anti-HCV C protein antiserum. Protein was detected after infection with a 1/750 virus dilution in HepG2 and CHO cells and 1/3750 virus dilution in BHK21 cells. Antisense experiments were conducted in HepG2 cells using a 1/100 virus dilution.

HCV C protein was decreased in the presence of HCV1. The inhibition was 50% at 2 µM and 0.4 µM HCV1. No consistent decrease was detected in randomer treated cells.

Additional oligonucleotides were also evaluated in this assay. HCV3 inhibited C protein production by about 60 to 70% at 0.4 µM; and HCV8 inhibited C protein production by about 40% at 2 µM and 0.4 µM.

In summary, the SFV/HCV recombinant provided a model system for HCV replication, and in a sequence specific inhibition of HCV protein expression was measured.

Some modified oligonucleotides were evaluated as luciferase inhibitors in HepG2 HCVLUC cells. Experiments were conducted with phosphorothioate oligodeoxynucleotides and with oligonucleotides having additional backbone modifications (chimeric and hybrid). In addition, the effects of oligonucleotide length on activity of modified backbones were also evaluated. The results of these experiments are shown in Table 5 below.

**TABLE 5**

| Oligonucleotide | Sequence | Modification | HepG2 HCVLUC (% control) at 0.2 µM^{a}) |
|---|---|---|---|
| HCV1 (SEQ ID NO:28) | 244-263 | PS | 46±18 |
| | | | |
| EG4-7 (SEQ ID NO:28) | 244-263 | 5'-(PO,2'OMe)₂₀-3' | 100 |
| | | | |
| EG4-10 (SEQ ID NO:28) | 244-263 | 5'-(PO)₁₅(PO,2'OMe)₅-3' | 99 |
| | | | |
| EG4-13 (SEQ ID NO:28) | 244-263 | 5'-(PO,2'OMe)₅-(PO)₁₀(PO,2'OMe)₅-3' | 86±2 |
| | | | |
| EG4-17 (SEQ ID NO:28) | 244-263 | 5'-(PS,2'OMe)₂₀-3' | 129±64 |
| | | | |
| EG4-20 (SEQ ID NO:28) | 244-263 | 5'-(PS)₁₅₋(PS,2'OMe)₅-3' | 48±27 |
| | | | |
| EG4-23 (SEQ ID NO:28) | 244-263 | 5'-(PS,2'OMe)₅-(PS)₁₀(PS,2'OMe)₅-3' | 57±20 |
| | | | |
| EG4-29 (SEQ ID NO:28) | 244-263 | 5'-(PS)₁₅(PO,2'OMe)₅-3' | 67±13 |
| | | | |
| EG-4-65 (SEQ ID NO:28) | 244-263 | 5'-(PO,2'OMe)₅-(PO)₁₀(PO,2'OMe)₅-3' | 82±11 |

| | | | |
|---|---|---|---|
| ^{a} - average ± standard deviation ^{b} - number of experiments | | | |

Hybrid oligonucleotides having SEQ ID NO:28 and having residues containing 2' OMe RNA at the 3' end or both ends, inhibited luciferase.

The most active modifications were five 2'OMe RNA phosphorothioate residues at the 3'end (EG4-20) or five 2'OMe RNA phosphorothioate residues at both ends (EG4-23). An oligonucleotide containing all 2'OMe phosphorothioate residues (EG4-17) did not inhibit luciferase. This suggests that RNase H is necessary for luciferase inhibition since 2'OMe residues are not substrates for RNase H. Hybrid oligonucleotides containing five 2'OMe phosphodiester residues at the 3' end (EG4-29) or five 2'OMe phosphodiester residues at both ends (EG4-65) were less active than their phosphorothioate counterparts. This suggests that phosphorothioate linkages are required for maximum activity.

Chimeric oligonucleotides can be prepared which contained phosphoramidate or methylphosphonate linkages in addition to phosphorothioate linkages. All sequences were based on HCV36 (SEQ ID NO:68) or HCV25 (SEQ ID NO:26). The results of luciferase inhibition studies using oligonucleotides having phosphorothioate and methylphosphonate linkages are shown below in Table 6.

**TABLE 6**

| Compound | Sequence | SEQID No. | Modification | HepG2 HCVLUC (%control) at 0.2µM^{a}) |
|---|---|---|---|---|
| HCV36 | 236-263 | 68 | PS | 56 |
| HCF36M | 236-263 | 68 | 5'-(PS)₂₂(PM)₅-3'^{a} | 54 |
| HCV36M2 | 236 -263 | 68 | 5'-(PS)₂PM(PS)₈PM (PS)₉PM(PS)₆PMPS-3' | 73 |
| HCV36M3 | 236-263 | 68 | 5'-(PS)₂PM₂(PS)₇PM (PS)₉PM(PS)₆(PM)₂PS-3' | 62 |
| HCV25 | 240-259 | 26 | PS | 42 |
| HCV25M | 240-259 | 26 | 5'-(PS)₁₄(PM)₅-3 | 75 |

| | | | | |
|---|---|---|---|---|
| ^{a} PM = P-Methyl | | | | |

In summary, antisense activity, as measured by luciferase inhibition, was retained in molecules with several backbone modifications: (1) oligonucleotides with phosphorothioate internucleotide linkages, (2) hybrids with DNA phosphorothioate internucleotide linkages and 2'-O-methyl RNA; (3) chimeric oligonucleotides having phosphorothioate and methylphosphonate internucleotide linkages. Chimeric oligonucleotides having phosphorothioate and PNBu internucleotide linkages and chimeric oligonucleotides having phosphorothioate and PNH(CH₂)₆NH³⁺ internucleotide linkages should also be effective. Antisense activity appeared to require phosphorothioate rather than phosphodiester backbones; longer chain lengths with chimeric oligonucleotides (that hybridize less strongly); and the ability to activate ribonuclease H.

The synthetic antisense oligonucleotides of the invention in the form of a therapeutic composition or formulation are useful in inhibiting HCV replication in a cell, and in treating hepatitis C viral infections and resulting conditions in an animal, such as chronic and acute hepatitis, hepatocellular carcinoma. They may be used on or as part of a pharmaceutical composition when combined with a physiologically and/or pharmaceutically acceptable carrier. The characteristics of the carrier will depend on the route of administration. Such a composition may contain, in addition to the synthetic oligonucleotide and carrier, diluents, fillers, salts, buffers, stabilizers, solubilizers, and other materials well known in the art. The pharmaceutical composition of the invention may also contain other active factors and/or agents which enhance inhibition of HCV expression. For example, combinations of synthetic oligonucleotides, each of which is directed to different regions of the HCV genomic or messenger RNA, may be used in the pharmaceutical compositions of the invention. The pharmaceutical composition of the invention may further contain other chemotherapeutic drugs. Such additional factors and/or agents may be included in the pharmaceutical composition to produce a synergistic effect with the synthetic oligonucleotide of the invention, or to minimize side-effects caused by the synthetic oligonucleotide of the invention. Conversely, the synthetic oligonucleotide of the invention may be included in formulations of a particular anti-HCV or anti-cancer factor and/or agent to minimize side effects of the anti-HCV factor and/or agent.

The pharmaceutical composition of the invention may be in the form of a liposome in which the synthetic oligonucleotides of the invention is combined, in addition to other pharmaceutically acceptable carriers, with amphipathic agents such as lipids which exist in aggregated form as micelles, insoluble monolayers, liquid crystals, or lamellar layers which are in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art, as disclosed, for example, in U.S. Patent No. 4,235,871; U.S. Patent No. 4,501,728; U.S. Patent No. 4,837,028; and U.S. Patent No. 4,737,323. The pharmaceutical composition of the invention may further include compounds such as cyclodextrins and the like which enhance delivery of oligonucleotides into cells , or such as slow release polymers.

As used herein, the term "therapeutically effective amount" or "therapeutic amount" means the total amount of each active component of the pharmaceutical composition or method that is sufficient to show a meaningful patient benefit, i.e., reduction in chronic or acute hepatitis or hepatocellular carcinoma. When applied to an individual active ingredient, administered alone, the term refers to that ingredient alone. When applied to a combination, the term refers to combined amounts of the active ingredients that result in the therapeutic effect, whether administered in combination, serially or simultaneously.

In practicing the use of the present invention, a therapeutically effective amount of one or more of the synthetic oligonucleotides of the invention is administered to a subject afflicted with an HCV-associated disease. The synthetic oligonucleotide of the invention may be administered in accordance with the method of the invention either alone of in combination with other known therapies for the HCV-associated disease. When co-administered with one or more other therapies, the synthetic oligonucleotide of the invention may be administered either simultaneously with the other treatment(s), or sequentially. If administered sequentially, the attending physician will decide on the appropriate sequence of administering the synthetic oligonucleotide of the invention in combination with the other therapy.

Administration of the synthetic oligonucleotide of the invention used in the pharmaceutical composition or to practice the method of treating an animal can be carried out in a variety of conventional ways, such as intraocular, oral ingestion, inhalation, or cutaneous, subcutaneous, intramuscular, or intravenous injection.

When a therapeutically effective amount of synthetic oligonucleotide of the invention is administered orally, the synthetic oligonucleotide will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% synthetic oligonucleotide and preferably from about 25 to 90% synthetic oligonucleotide. When administered in liquid form, a liquid carrier such as water, petroleum, oils of animal or plant origin such as peanut oil, mineral oil, soybean oil, sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of the synthetic oligonucleotide and preferably from about 1 to 50% synthetic oligonucleotide.

When a therapeutically effective amount of synthetic oligonucleotide of the invention is administered by intravenous, cutaneous or subcutaneous injection, the synthetic oligonucleotide will be in the form of a pyrogen-free, parenterally acceptable aqueous solution. The preparation of such parenterally acceptable solutions, having due regard to pH, isotonicity, stability, and the like, is within the skill in the art. A preferred pharmaceutical composition for intravenous, cutaneous, or subcutaneous injection should contain, in addition to the synthetic oligonucleotide, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The amount of synthetic oligonucleotide in the pharmaceutical composition of the present invention will depend upon the nature and severity of the condition being treated, and on the nature of prior treatments which the patient has undergone. Ultimately, the attending physician will decide the amount of synthetic oligonucleotide with which to treat each individual patient. Initially, the attending physician will administer low doses of the synthetic oligonucleotide and observe the patient's response. Larger doses of synthetic oligonucleotide may be administered until the optimal therapeutic effect is obtained for the patient, and at that point the dosage is not increased further. It is contemplated that the various pharmaceutical compositions used to practice the method of the present invention should contain about 1.0 ng to about 2.5 mg of synthetic oligonucleotide per kg body weight.

The duration of intravenous therapy using the pharmaceutical composition of the present invention will vary, depending on the severity of the disease being treated and the condition and potential idiosyncratic response of each individual patient. It is contemplated that the duration of each application of the synthetic oligonucleotide will be in the range of 12 to 24 hours of continuous intravenous administration. Ultimately the attending physician will decide on the appropriate duration of intravenous therapy using the pharmaceutical composition of the present invention.

The invention also provides kits for inhibiting hepatitis C virus replication and infection in a cell. Such a kit includes a synthetic oligonucleotide specific for HCV genomic or messenger RNA, such as those described herein. For example, the kit may include at least one of the non-contiguous oligonucleotides having SEQ ID NO: 38, 39, 40, 41, 42, 43, 44, 45, 46, 47, 48, 49, 50, 51, 52, 53, 54, 55, 56, 57, 58, 59, 60, 61, 62, 63, 64, 65 66, and 67 and/or those oligonucleotides having SEQ ID NOS: 134-158 and 166-170 and listed in Tables 1C-1E. These oligonucleotides may have modified backbones, such as those described above, and may be RNA/DNA hybrids containing, for example, at least one 2'-O-methyl. The kit of the invention may optionally include buffers, cell or tissue preparation reagents, cell or tissue preparation tools, vials, and the like.

In another aspect, the invention provides a method of detecting the presence of HCV in a sample, such as a solution or biological sample. In this method, the sample is contacted with a synthetic oligonucleotide of the invention. Hybridization of the oligonucleotide to the HCV nucleic acid is then detected if the- HCV is present in the sample.

Another aspect of the invention are kits for detecting HCV in a sample. Such kits include a non-contiguous synthetic oligonucleotide of the invention, and means for detecting the oligonucleotide hybridized with the nucleic acid.

The following examples illustrate the preferred modes of making and practicing the present invention, but are not meant to limit the scope of the invention since alternative methods may be utilized to obtain similar results. The assays relating to contiguous oligonucleotides are only comparative examples.

### EXAMPLES

### 1. Oligonucleotide Synthesis

Oligonucleotides were synthesized using standard phosphoramidite chemistry (Beaucage (1993) Meth. Mol. Biol. 20:33-61; Uhlmann et al. (1990) Chem. Rev, 90:543-584) on either an ABI 394 DNA/RNA synthesizer (Perkin-Elmer, Foster City, CA), a Pharmacia Gene Assembler Plus (Pharmacia, Uppsala, Sweden) or a Gene Assembler Special (Pharmacia, Uppsala, Sweden) using the manufacturers standard protocols and custom methods. The custom methods served to increase the coupling time from 1.5 min to 12 min for the 2'-OMe RNA amidites. The Pharmacia synthesizers required additional drying of the amidites, activating reagent and acetonitrile. This was achieved by the addition of 3 Å molecular sieves (EM Science, Gibbstown, NJ) before installation on the machine.

DNA β-cyanoethyl phosphoramidites were purchased from Cruachem (Glasgow, Scotland). The DNA support was 500 Å pore size controlled pore glass (CPG) (PerSeptive Biosystems, Cambridge, MA) derivatized with the appropriate 3' base with a loading of between 30 to 40 mmole per gram. 2'-OMe RNA β-cyanoethyl phosphoramidites and CPG supports (500 A) were purchased from Glen Research (Sterling, VA). For synthesis of random sequences, the DNA phosphoramidites were mixed by the synthesizer according to the manufacturer's protocol (Pharmacia, Uppsala, Sweden).

All 2'-OMe RNA-containing oligonucleotides were synthesized using ethylthiotetrazole (American International Chemical (AIC), Natick, MA) as the activating agent, dissolved to 0.25 M with low water acetonitrile (Aldrich, Milwaukee, WI). Some of the DNA-only syntheses were done using 0.25 M ethylthiotetrazole, but most were done using 0.5 M 1-H-tetrazole (AIC). The thiosulfonating reagent used in all the PS oligonucleotides was 3H-1,2-benzodithiol-3-one 1,1-dioxide (Beaucage Reagent) (R.I. Chemical, Orange, CA, or AIC, Natick, MA) as a 2% solution in low water acetonitrile (w/v).

After completion of synthesis, the CPG was air dried and transferred to a 2 ml screw-cap microfuge tube. The oligonucleotide was deprotected and cleaved from the CPG with 2 ml ammonium hydroxide (25-30%). The tube was capped and incubated at room temperature for greater than 20 minutes, then incubated at 55°C for greater than 7 hours. After deprotection was completed, the tubes were removed from the heat block and allowed to cool to room temperature. The caps were removed and the tubes were microcentrifuged at 10,000 rpm for 30 minutes to remove most of the ammonium hydroxide. The liquid was then transferred to a new 2 ml screw cap microcentrifuge tube and lyophilized on a Savant speed vac (Savant, Farmingdale, NY). After drying, the residue was dissolved in 400 µl of 0.3 M NaCl and the DNA was precipitated with 1.6 ml of absolute EtOH. The DNA was pelleted by centrifugation at 14,000 rpm for 15 minutes, the supernatant decanted, and the pellet dried. The DNA was precipitated again from 0.1 M NaCl as described above. The final pellet was dissolved in 500 µl H₂O and centrifuged at 14,000 rpm for 10 minutes to remove any solid material. The supernatant was transferred to another microcentrifuge tube and the amount of DNA was determined spectrophotometrically. The concentration was determined by the optical density at 260 nM. The E₂₆₀ for the DNA portion of the oligonucleotide was calculated by using OLIGSOL (Lautenberger (1991) Biotechniques 10:778-780). The E₂₆₀ of the 2'-OMe portion was calculated by using OLIGO 4.0 Primer Extension Software (NBI, Plymouth, MN).

Oligonucleotide purity was checked by polyacrylamide gel electrophoresis (PAGE) and UV shadowing. 0.2 OD₂₆₀ units were loaded with 95% formamide/H₂O and Orange G dye onto a 20% denaturing polyacrylamide gel (20 cm x 20 cm). The gel was run until the Orange G dye was within one inch of the bottom of the gel. The band was visualized by shadowing with shortwave UV light on a Keiselgel 60 F254 thin layer chromatography plate (EM Separations, Gibbstown, NJ).

### 2. Synthesis and Purification of Oligonucleotides Containing Mixed Backbones

Standard phosphoramidite chemistry was applied in the synthesis of oligonucleotides containing methylphosphonate linkages using two Pharmacia Gene Assembler Special DNA synthesizers. One synthesizer was used for the synthesis of phosphorothioate portions of oligonucleotides using β-cyanoethyl phosphoramidites method discussed above. The other synthesizer was used for introduction of methylphosphonate portions. Reagents and synthesis cycles that had been shown advantageous in methylphosphonate synthesis were applied (Hogrefe et al., in Methods in Molecular Biology, Vol. 20: Protocols for Oligonucleotides and Analogs (Agrawal, ed.) (1993) Humana Press Inc., Totowa, NJ). For example, 0.1 M methyl phosphonamidites (Glen Research) were activated by 0.25 M ethylthiotetrazole; 12 minute coupling time was used; oxidation with iodine (0.1 M) in tetrahydrofuran/2,6-lutidine/water (74.75/25/0.25) was applied immediately after coupling step; dimethylaminopyridine (DMAP) was used for capping procedure to replace standard N-methylimidazole (NMI). The chemicals were purchased from Aldrich (Milwaukee, WI).

The work up procedure was based on a published procedure (Hogrefe et al. (1993) Nucleic Acids Research 21:2031-2038). The product was cleaved from the resin by incubation with 1 ml of ethanol/acetonitrile/ammonia hydroxide (45/45/10) for 30 minutes at room temperature. Ethylenediamine (1.0 ml) was then added to the mixture to deprotect at room temperature for 4.5 hours. The resulting solution and two washes of the resin with 1 ml 50/50 acetonitrile/0.1 M triethylammonium bicarbonate (TEAB), pH 8, were pooled and mixed well. The resulting mixture was cooled on ice and neutralized to pH 7 with 6 N HCl in 20/80 acetonitrile/water (4-5 ml), then concentrated to dryness using the Speed Vac concentrater. The resulting solid residue was dissolved in 20 ml of water, and the sample desalted by using a Sep-Pak cartridge. After passing the aqueous solution through the cartridge twice at a rate of 2 ml per minute, the cartridge was washed with 20 ml 0.1 M TEAB and the product eluted with 4 ml 50% acetonitrile in 0.1 M TEAB at 2 ml per minute. The eluate was evaporated to dryness by Speed Vac. The crude product was purified by the PAGE procedure, desalted using a Sep-Pak cartridge, then exchanged counter ion into sodium by ethanol precipitation of NaCl solutions, as described above. The product was dissolved in 400 ml water and quantified by UV absorbance at 260 nM.

### 3. Constructs

The oligonucleotide constructs which were used are shown schematically in FIG. 9. The HCV-luciferase fusion protein (HCVLUC) contained bases 52 to 338 of HCV sequence. HCV sequences 52-337 (Kato et al. (1990) Proc. Natl. Acad. Sci. (USA) 87:9524) were subcloned from plasmid pHO3-65 (Hoffmann-La Roche, Basel, Switzerland) using PCR. The 5' primer was a T7 primer which is upstream of the HCV region in pHO3-65. The 3' PCR primer contained bases complementary to luciferase and 18 bases complementary to HCV. The PCR product was subcloned into pCRII (Invitrogen, San Diego, CA). The correct sequence confirmed and then cloned into pGEMluc (Promega, Madison, WI). This fused HCV sequences to luciferase, substituting the first 9 bases of HCV for the first 6 bases of luciferase to make pGEMHCVLUC. HCVLUC sequences were subcloned into pcDNAIneo (Invitrogen, San Diego, CA) to produce pcHCVLUCneo for stable expression in mammalian cells.

HCV sequences 52-337 and 254-1417 (Kato et al. (1990) Proc. Natl. Acad. Sci. (USA) 87:9524) from pHO3-65 and pHO3-62 (Hoffmann-La Roche, Basel, Switzerland), respectively, were subcloned together into pBluescriptIISK (Stratagene, La Jolla, CA) to produce HCV sequences 52-1417 in a single vector. HCV52-1417 was then subcloned into pcDNAIneo (Invitrogen, San Diego, CA) to produce pcHCVneo.

### 4. RNase H Assays

### A. Plasmid Preparation

The pcHCVneo plasmid (10 µg) was linearized with Xbal restriction enzyme (New England Biolabs, Beverly, MA, 20 U) for 2 hours at 37°C, treated with proteinase K (Stratagene, La Jolla, CA) (0.1 µg/µl) for 1 hour at 37°C and twice phenol/chloroform extracted. The linearized plasmid was ethanol precipitated and isolated from the supernatant by centrifugation. The dried pellet was dissolved in diethylpyrocarbonate (DRPC) (Aldrich, Milwaukee, WI)-treated water to a concentration of 0.5 µg/µl.

### B. In Vitro Transcription and ³²P-Labelling of HCV mRNA

HCV mRNA was transcribed in vitro using either the Stratagene mRNA Transcription Kit (La Jolla, CA) or the Ambion MEGAscript In vitro Transcription Kit (Austin, TX), and each manufacturers T7 RNA polymerase supplied with each kit. Transcription was performed in the presence of 7.5 mM CTP, 7.5 mM ATP, 75 mM UTP, 6 mM GTP, and 6 mM guanosine hydrate. The reduced GTP concentration allowed the initiation of a high percentage of the transcripts with guanosine to facilitate end-labelling of the mRNA without pretreatment with alkaline phosphatase. After transcribing for 3 hours at 37YC, the reaction was treated with RNase-free DNase (Stratagene, La Jolla, CA or Ambion, Austin, TX), twice phenol/chloroform extracted, and chromatographed through a G-50 Sephadex spin-column (Boehringer-Mannheim, Indianapolis, IN or Pharmacia, Uppsala, Sweden) to remove unreacted nucleotides and nucleoside. The recovered mRNA was quantitated by measuring the UV absorbance at 260 nm using an extinction coefficient of 10000 M⁻¹ cm⁻¹ base⁻¹ of the mRNA.

Yields were generally 200-250 µg RNA/µg DNA from a 20 µl reaction. The mRNA was aliquotted (15 µg) and stored at -80°C until needed. The mRNA (15 µg) was end-labelled with 20-25 units of T4 polynucleotide kinase (Pharmacia, Uppsala, Sweden) and 50 µCi [γ-³²P]ATP (Amersham, Arlington Heights, IL), 6000 Ci/mmol). The labelled mRNA was purified by chromatography through a G-50 Sephadex spin column (Boehringer-Mannheim, Indianapolis, IN, or Pharmacia, Uppsala, Sweden).

### C RNase H Cleavage with Random 20mer Library

End-labelled RNA (20-100 nM) was incubated with a 20 base random DNA library (50-100 µM) (synthesized on Pharmacia Gene Assembler; all oligonucleotide synthesis, above), boiled previously to dissociate any aggregates, for 90 min at 37°C in 9 µl 1x buffer (40 mM Tris-HCl pH 7.4, 4 mM MgCl₂, 1 mM DTT). RNase H (Boehringer-Mannheim, Indianapolis, IN) (1 µl, 1 unit/µl) was then added. The reaction was incubated at 37°C for 10 min, quenched by addition of 10 µl 90% formamide containing 0.1% phenol red/0.1% xylene cyanol, and frozen on dry ice. The quenched reactions were boiled for 2.5 to 3 minutes, quenched on ice, and 5 to 7 µl loaded onto a denaturing 4% polyacrylamide gel prerun to 50 to 55°C. The phenol red was typically run to the bottom of the gel, which was then dried at 80°C under vacuum. The gel was autoradiographed using XOMAT film (Kodak, Rochester, NY) or analyzed using phosphorimage technology on a Molecular Dynamics (Sunnyvale, CA) or Bio Rad Phosphorimager (Hercules, CA).

### D. Cleavage of HCV mRNA with Specific Antisense Oligonucleotides

In 9 µl 1x RNase H buffer (40 mM Tris-HCl pH 7.4, 4 mM MgCl₂, 1 mM DTT), 20-100 nM [5'-³²P]-labelled mRNA and 100 nM oligonucleotides (ODN) were preincubated for 15 min at 37°C. 1 µl RNase H (1 U/µl) was added, and the reaction was incubated at 37°C for 10 min. The reactions were quenched and analyzed as described above. Quantitation of the cleavage products was performed using software supplied with the PhosphorImager (Molecular Dynamics, Sunnyvale, CA, or Bio-Rad Laboratories, Hercules, CA). "Counts" were determined by drawing a box around the band of interest and subtracting the background determined with a box drawn nearby. Counts in a product band were compared to total counts in the lane above that band to determine % cleavage. This accounts for the cleavage of small amounts of incomplete transcripts.

### E. Cleavage of HCV mRNA with Semirandom Oligonucleotides

Semirandom oligonucleotides (100 µM in H₂O) were boiled for 1 min to dissociate any aggregates formed between complementary sequences in the mix and 1 µl (final concentration 10 µM) was added to 8 µl 1x RNase H buffer (40 mM Tris-HCl pH 7.4, 4 mM MgCl₂, 1 mM DTT) containing labelled mRNA (20-100 nM). After a 15 minute preincubation at 37°C, RNase H was added (1 U) and incubated for 10 min at 37°C. The reactions were quenched and analyzed as described above. Sites of cleavage were estimated using DNA and/or RNA molecular size markers.

### 5. Inhibition of HCV-Luciferase Fusion Protein Expression in Stably Transfected Cells

### A. Transfection

HepG2 cells (ATCC HB8065, American Type Culture Collection, Rockville, MD) were maintained in DMEM with 10% fetal calf serum. Cells were transfected with pcHCVLUCneo by the calcium phosphate procedure (Sambrook et al. (1989) Molecular Cloning, A Laboratory Manual (2nd ed.), Cold Spring Harbor Laboratory Press, pp. 16.30-16.40). Stably transfected clones were selected with (0.75 µg/ml) Geneticin (Gibco/BRL, Gaithersburg, MD). Clones were evaluated for luciferase expression as described below. A similar luciferase construct lacking HCV sequence was also expressed stably in HepG2 cells.

Cells were incubated in lysis buffer (Analytical Luminescence Laboratory, San Diego, CA). Cell lysate (20 µl) was transferred to a White Microlite Plate (Dynatech Laboratories, Chantilly, VA) and 50 µl substrate A (Analytical Luminescence Laboratory, San Diego, CA) was added to the plate. Luciferase activity was measured in a Microplate Luminometer LB96P (EG&G Berthold, Nashua, NH) by injecting 50 µl Substrate B (Analytical Luminescence Laboratory, San Diego, CA)), waiting 2 seconds, and then integrating the luminescence signal over 10 seconds.

### B. Inhibition of HCVLUC Expression

HepG2 HCVLUC cells were seeded onto a 96 well plate (5000 cells/well), and incubated overnight at 37°C. Oligonucleotides were diluted in Optimem (Gibco/BRL, Gaithersburg, MD) containing 10 µg/ml Lipofectin (Gibco/BRL, Gaithersburg, MD). Medium was removed from cells and replaced with 100 µl oligonucleotide in Optimem/Lipofectin. Cells were incubated overnight, washed twice with PBS, and then luciferase expression was evaluated.

Alternatively, stably transfected HepG2 cells were treated with oligonucleotides as described previously, except that oligonucleotides were mixed with 4ug/ml Cellfectin (Gibco-BRL). Inhibition was measured at four oligonucleotide concentrations, relative to cells treated only with Cellfectin. EC₅₀ was determined from graphs of the dose response curves. Most active compounds contained 5x5 and 6x6 2'OMe. When more than 12 2'OMe residues were present, oligonucleotides were less active. In this assay, when 18 or 20 2'OMe residues were present (9x) or all 2'OMe) HCVLUC was not inhibitied at any concentration tested (up to 1 uM). The results are shown below in Table 7.

**Table 7**

| **Sequence** | **SEQ ID No.** | **Backbone** | **EC**_{**50**} **µM** |
|---|---|---|---|
| HCV1 | 28 | PS | 0.04 |
| HCV1 | 28 | 5x5 2'OMe PS | 0.02 |
| HCV1 | 28 | 6x6 2'OMe PS | 0.03 |
| HCV1 | 28 | 7x7 2'OMe PS | 0.09 |
| HCV1 | 28 | 8x8 2'OMe PS | 0.07 |
| HCV1 | 28 | 9x5 2'OMe PS | 0.08 |
| HCV1 | 28 | 5x9 2'OMe PS | 0.05 |
| HCV1 | 28 | 3x11 2'OMe PS | 0.09 |
| HCV1 | 28 | 11x3 2'OMe PS | 0.2 |
| HCV1 | 28 | 0x14 2'OMe PS | 0.4 |

All oligonucleotide-treated samples were measured in triplicate wells. Untreated control samples were measured in 12 wells. Data was evaluated as % control (treated sample/untreated sample x 100) for each oligonucleotide.

### 6. Inhibition of HCV RNA Expression in Stably Transfected Cells

Cells were transfected with pcHCVneo, and cells stably expressing HCV C protein were selected by Western blot using a rabbit polyclonal antiserum specific for HCV protein (Hoffmann-La Roche, Basel, Switzerland). Cells also expressed HCV RNA as detected by ribonuclease protection assay using probes specific for the 5' UTR and HCV C protein coding sequence.

A ribonuclease protection assay was used to measure HCV RNA in HepG2 cells stably transfected with pcHCVneo. HCV specific riboprobes were prepared which included HCV sequences 52 to 338 (probe 1) or 238 to 674 (probe 2). HepG2 HCV cells (1 x 10⁶ cells) were seeded into 100 mm dishes, incubated overnight, then treated with oligonucleotide in the presence of 10 µg/ml Lipofectin for 4 hours as described above. Cells were incubated overnight. Total RNA was isolated using Trizol (Gibco/BRL, Gaithersburg, MD) according to the manufacturer's instructions.

Ribonuclease protection assays were performed using 10 µg of RNA. RNA was hybridized with radiolabelled probe overnight and then digested with single-strand specific RNases A and T1 (RPAII kit, Ambion, Austin, TX) according to the manufacturer's instructions. Ribonuclease digestion products were separated on a 6% polyacrylamide/urea gel. The gel was dried and exposed to x-ray film overnight. Molecular weights were estimated by comparison to RNA standards electrophoresed on the same gel (Ambion, Austin, TX). In addition, amounts of RNA were quantitated on a phosphorimager (BioRad GS250, Hercules, CA).

### 7. Inhibition of Protein Expression in SFV/HCV Infected Cells

HCV bases 1-2545 were used to generate a recombinant virus with Semliki Forest virus (SFV/HCV) (Gibco/BRL, Gaithersburg, MD). HCV sequences were subcloned from vv1-2545 (Hoffmann-La Roche, Basel, Switzerland) into pSFV1. SFV/HCV sequences were transcribed in vitro using SP6 RNA polymerase. RNA was also transcribed from pSFV2-Helper (Gibco/BRL, Gaithersburg, MD) which provided SFV structural proteins to the recombinant virus. The two RNAs were co-transfected into BHK21 cells (ATCC Ac. No. CCL 10, American Type Culture Collection, Rockville, MD), according to the manufacturer's instructions (SFV Gene Expression System, Gibco/BRL, Gaithersburg, MD.) to generate the recombinant virus. Supernatant was removed from the cultures 48 hours post-transfection and used as a virus stock for subsequent experiments. pSFV2-Helper produces a structural protein (p62) containing an eight base mutation, converting three arginines to non-basic amino acids. This modification renders the recombinant virus non-infectious unless the p62 protein is first digested with chymotrypsin (Gibco/BRL, Gaithersburg, MD). Recombinant virus required chymotrypsin activation before infection.

HepG2 cells (10⁵ cells/well in a 6 well dish) were pretreated for 4 hours with different concentrations of oligonucleotide in the presence of 10 µg/ml Lipofectin in Optimem. Oligonucleotide was then removed, and cells were infected with chymotrypsin activated SFV/HCV (diluted 1/100 in PBS with Ca²⁺, Mg²⁺) for 1 hour at 37°C. The inoculum was removed, oligonucleotide in Optimum was added to cells, and cells were incubated overnight at 37°C. Cells were then lysed, protein was quantitated and equal amounts of protein were electrophoresed on an SDS/polyacrylamide gel. Protein was detected by Western blotting. The blots were scanned with a flat bed scanner (Umax Data Systems Inc., Hsinchu, Taiwan, ROC) and quantitated with densitometric software (Scan Analysis Biosoft, Ferguson, MO).

Alternatively, SFV/HCV virus stocks were prepared as described previously. SFV/HCV inhibition was measured as described previously except that, in some experiments, HepG2 cells were infected with SFV/HCV virus for one hour at 37°C, virus incolum was removed, and then oligonucleotide was added in the presence of lipofectin. In some experiments, cells were not incubated in the presence of oligonucleotide before infection. That oligonucleotides of the invention inhibited HCV C protein production in this assay system is shown below in Table 8.

**Table 8**

| **Sequence** | **SEQ ID No.** | **Backbone** | **≥40% Inhibition at 2, 0.4µM** |
|---|---|---|---|
| HCV1 | 28 | PS | yes |
| HCV3 | 35 | PS | yes |
| HCV1 (EG4-20) | 28 | 0x5 2'OMe PS | yes |
| HCV1 (EG4-23) | 28 | 5x5 2'OMe PS | yes |
| HCV1 | 28 | 6x6 2'OMe PS | yes |
| HCV1 | 28 | 3x11 2'OMe PS | yes |
| HCV1 (EG4-29) | 28 | 0x5 2'OMe PS | yes |
| HCV8 | 9 | PS | yes |
| HCV28 | 30 | PS | yes |
| HCV45 | 23 | PS | yes |

The contiguos oligonucleotides shown in sequence protocoll are only for illustrative purposes.

## Claims

1. A synthetic oligonucleotide comprising a sequence complementary to a sequence consisting of two non-contiguous regions of an HCV messenger or genomic RNA.

2. A synthetic oligonucleotide comprising a sequence complementary to a sequence consisting of three non-contiguous regions of an HCV messenger or genomic RNA.

3. A synthetic oligonucleotide according to claim 1 or 2 wherein one of the non-contiguous regions is the 5 untranslated region.

4. An oligonucleotide according to claim 1 having 18 to 24 nucleotides.

5. An oligonucleotide according to claim 1 or 2 wherein one portion of the oligonucleotide has the sequence GGGGUCCUGGAG (SEQ ID NO: 47) or has the sequence CAACACUACUCG.

6. A synthetic oligonucleotide according to any one of claims 1-5 which is modified.

7. An oligonucleotide according to claim 6 wherein the modification comprises at least one internucleotide linkage selected from the group consisting of alkylphosphonate, phosphorothioate, phosphorodithioate, alkylphosphonothioate, phosphoramidate, carbamate, carbonate, phosphate triester, acetamidate, carboxymethyl ester, and combinations thereof.

8. An oligonucleotide according to claim 7 comprising at least one phosphorothioate internucleotide linkage.

9. An oligonucleotide according to claim 7 wherein the internucleotide linkages in the oligonucleotide are phosphorothioate internucleotide linkages.

10. An oligonucleotide according to claim 6 which comprises at least on deoxyribonucleotide.

11. An oligonucleotide according to claim 6 which comprises at least one ribonucleotide.

12. An oligonucleotide according to claim 10 which additionally comprises at least one ribonucleotide.

13. An oligonucleotide according to claim 12 wherein an oligodeoxyribonucleotide region is interposed between two oligoribonucleotide regions, or the inverted configuration thereof.

14. An oligonucleotide according to any one of claims 11-13 wherein the ribonucleotide is a 2'-O-methyl ribonucleotide.

15. An oligonucleotide according to claim 12 which comprises at least one 2'-O-methyl ribonucleotide at the 3'-end of the oligonucleotide.

16. An oligonucleotide according to claim 15 which further comprises at least one 2'-O-methyl ribonucleotide at the 5'-end of the oligonucleotide.

17. An oligonucleotide according to claim 1 or 2 comprising a sequence selected from the group consisting of SEQ ID NOS: 38, 39, 40, 41, 42, 43, 44, 45, 46, 48, 49, 50, 51, 52, 53, 54, 55, 56, 57, 58, 59, 60, 61, 62, 63, 64, 65, 66, and 67, as set forth in Table 2.

18. An oligonucleotide according to claim 1 or 2 comprising a sequence selected form the group consisting of SEQ ID NOS: 134, 135, 136, 137, 138, 139, 140, 141, 142, 143, 144, 145, 146, and 147, as set forth in Table 1C.

19. An oligonucleotide according to claim 1 or 2 comprising a sequence selected from the group consisting of SEQ ID NOS: 148, 149, 150, 151, 152, 153, 154, 155, 156, 157, and 158, as set forth in Table 1D.

20. An oligonucleotide according to claim 6 which oligonucleotide is self stabilized by a loop.

21. An oligonucleotide according to claim 6 wherein the modification is selected from the group consisting of a nicked dumbbell, a closed dumbbell, 2', 3' and/or 5' caps, additions to the molecule at the internucleotide phosphate linkage, oxidation, oxidation/reduction, and oxidation/reductive amination, including combination thereof.

22. An oligonucleotide according to claim 6 wherein at least one nucleoside is substituted by inosine or wherein at least one deoxycytosine is substituted by 5-methyl deoxycytosine.

23. An oligonucleotide according to claim 6 wherein the oligonucleotide is modified by incorporating at least one additional triplex forming strand.

24. An oligonucleotide according to claim 23 having a nucleotide sequence selected from the group consisting of SEQ ID NOS: 166, 167, 168, 169, and 170, as set forth in Table 1E.

25. A pharmaceutical composition comprising at least one oligonucleotide according to any one of claims 1-24 and a pharmaceutically acceptable carrier.

26. A pharmaceutical composition comprising at least two different oligonucleotides according to any one of claims 1-24.

27. An oligonucleotide according to any one of claims 1-24, for use as a therapeutically active compound, especially for use in the control or prevention of hepatitis C virus infection or replication.

28. Use of an oligonucleotide according to any one of claims 1-24 for the preparation of a pharmaceutic composition for inhibiting hepatitis C virus replication in a cell or for treating hepatitis C virus infection.

29. A method of defecting the presence of HCV in a sample, comprising the steps of:
(a) contacting the sample with a synthetic oligonucleotide according to claim 1; and
(b) detecting the hybridization of the oligonucleotide to the nucleic acid.

30. A kit for the detection of HCV in a sample comprising:
(a) a synthetic oligonucleotide according to claim 1; and
(b) means for detecting the oligonucleotide hybridized with the nucleic acid.

## Patentansprüche

1. Ein synthetisches Oligonukleotid, umfassend eine Sequenz; die komplementär zu einer Sequenz ist, die aus zwei nicht-zusammenhängenden Bereichen einer Messengeroder genomischen RNA von HCV besteht.

2. Ein synthetisches Oligonukleotid, umfassend eine Sequenz, die komplementär zu einer Sequenz ist, die aus drei nicht-zusammenhängenden Bereichen einer Messengeroder genomischen RNA von HCV besteht.

3. Ein synthetisches Oligonukleotid gemäß Anspruch 1 oder 2, wobei einer der nicht-zusammenhängenden Bereiche der 5'-untranslatierte Bereich ist.

4. Ein Oligonukleotid gemäß Anspruch 1 mit 18 bis 24 Nukleotiden.

5. Ein Oligonukleotid gemäß Anspruch 1 oder 2, wobei ein Teil des Oligonukleotids die Sequenz GGGGUCCUGGAG (SEQ ID NO:47) oder die Sequenz CAACACUACUCG besitzt.

6. Ein synthetisches Oligonukleotid gemäß einem der Ansprüche 1-5, das modifiziert ist.

7. Ein Oligonukleotid gemäß Anspruch 6, wobei die Modifikation wenigstens eine Intemukleotidbindung umfasst, ausgewählt aus der Gruppe bestehend aus Alkylphosphonat, Phosphorthioat, Phosphordithioat, Alkylphosphonothioat, Phosphoramidat, Carbamat, Carbonat, Phosphat-Triester, Acetamidat, Carboxymethylester und Kombinationen davon.

8. Ein Oligonukleotid gemäß Anspruch 7, umfassend wenigstens eine Phosphorthioat-Intemukleotidbindung.

9. Ein Oligonukleotid gemäß Anspruch 7, wobei die Intemukleotidbindungen in dem Oligonukleotid Phosphorthioat-lntemukleotidbindungen sind.

10. Ein Oligonukleotid gemäß Anspruch 6, das wenigstens ein Desoxyribonukleotid umfasst.

11. Ein Oligonukleotid gemäß Anspruch 6, das wenigstens ein Ribonukleotid umfasst.

12. Ein Oligonukleotid gemäß Anspruch 10, das zusätzlich wenigstens ein Ribonukleotid umfasst.

13. Ein Oligonukleotid gemäß Anspruch 12, wobei zwischen zwei Oligoribonukleotid-Bereiche oder der umgekehrten Konfiguration davon ein Oligodesoxyribonukleotid-Bereich zwischengeschaltet ist.

14. Ein Oligonukleotid gemäß einem der Ansprüche 11-13, wobei das Ribonukleotid ein 2'-O-methyl-Ribonukleotid ist.

15. Ein Oligonukleotid gemäß Anspruch 12, umfassend wenigstens ein 2'-O-methyl-Ribonukleotid am 3'-Ende des Oligonukleotids.

16. Ein Oligonukleotid gemäß Anspruch 15, umfassend weiterhin wenigstens ein 2'-O-methyl-Ribonukleotid am 5'-Ende des Oligonukleotids.

17. Ein Oligonukleotid gemäß Anspruch 1 oder 2, umfassend eine Sequenz ausgewählt aus der Gruppe bestehend aus SEQ ID NO: 38, 39, 40, 41, 42, 43, 44, 45, 46, 48, 49, 50, 51, 52, 53, 54, 55, 56, 57, 58, 59, 60, 61, 62, 63, 64, 65, 66 und 67, wie in Tabelle 2 dargestellt.

18. Ein Oligonukleotid gemäß Anspruch 1 oder 2, umfassend eine Sequenz ausgewählt aus der Gruppe bestehend aus SEQ ID NO: 134, 135, 136, 137, 138, 139, 140, 141, 142, 143, 144, 145, 146 und 147, wie in Tabelle 1C dargestellt.

19. Ein Oligonukleotid gemäß Anspruch 1 oder 2, umfassend eine Sequenz ausgewählt aus der Gruppe bestehend aus SEQ ID NO: 148, 149, 150, 151, 152, 153, 154, 155, 156, 157 und 158, wie in Tabelle 1D dargestellt.

20. Ein Oligonukleotid gemäß Anspruch 6, wobei das Oligonukleotid durch eine Schleife selbst-stabilisiert ist.

21. Ein Oligonukleotid gemäß Anspruch 6, wobei die Modifikation ausgewählt ist aus der Gruppe bestehend aus einer gekerbten Hantel ("nicked dumbbell"), einer geschlossenen Hantel ("closed dumbbell"), 2'-, 3-' und/oder 5'-Caps, Additionen an das Molekül an der Intemukleotid-Phosphatbindung, Oxidation, Oxidation/Reduktion, und Oxidation/reduktive Aminierung, einschließlich Kombinationen davon.

22. Ein Oligonukleotid gemäß Anspruch 6, wobei wenigstens ein Nukleosid durch Inosin substituiert ist oder wobei wenigstens ein Desoxycytosin durch 5-methyl-Desoxycytosin substituiert ist.

23. Ein Oligonukleotid gemäß Anspruch 6, wobei das Oligonukleotid durch Einbau von wenigstens einem Triplex-bildenden Strang modifiziert ist.

24. Ein Oligonukleotid gemäß Anspruch 23 mit einer Nukleotidsequenz ausgewählt aus der Gruppe bestehend aus SEQ ID NO: 166, 167, 168, 169 und 170, wie in Tabelle 1E dargestellt.

25. Eine pharmazeutische Zusammensetzung, umfassend wenigstens ein Oligonukleotid gemäß einem der Ansprüche 1-24 und einen pharmazeutisch verträglichen Träger.

26. Eine pharmazeutische Zusammensetzung, umfassend wenigstens zwei verschiedene Oligonukleotide gemäß einem der Ansprüche 1-24.

27. Ein Oligonukleotid gemäß einem der Ansprüche 1-24 zur Verwendung als eine therapeutisch aktive Verbindung, insbesondere zur Verwendung bei der Kontrolle oder Prävention der Hepatitis C Virus-Infektion oder -Replikation.

28. Verwendung eines Oligonukleotids gemäß einem der Ansprüche 1-24 zur Zubereitung einer pharmazeutischen Zusammensetzung zur Hemmung der Replikation von Hepatitis Virus C in einer Zelle oder zur Behandlung von Infektion mit Hepatitis Virus C.

29. Ein Verfahren zum Nachweis von HCV in einer Probe, umfassend die Schritte:
(a) In-Kontakt-bringen der Probe mit einem synthetischen Oligonukleotid gemäß Anspruch 1, und
(b) Nachweisen der Hybridisierung des Oligonukleotids an die Nukleinsäure.

30. Ein Kit zum Nachweis von HCV in einer Probe, umfassend:
(a) ein synthetisches Oligonukleotid gemäß Anspruch 1; und
(b) Mittel zum Nachweisen des an die Nukleinsäure hybridisierten Oligonukleotids.

## Revendications

1. Oligonucléotide synthétique comprenant une séquence complémentaire d'une séquence consistant en deux régions non-contiguës d'un ARN messager ou génomique de VHC.

2. Oligonucléotide synthétique comprenant une séquence complémentaire d'une séquence consistant en trois régions non-contiguës d'un ARN messager ou génomique de VHC.

3. Oligonucléotide synthétique selon la revendication 1 ou 2, dans lequel l'une des régions non-contiguës est la région 5' non-traduite.

4. Oligonucléotide selon la revendication 1 ayant 18 à 24 nucléotides.

5. Oligonucléotide selon la revendication 1 ou 2, dans lequel une portion de l'oligonucléotide a la séquence GGGGUCCUGGAG (SEQ ID NO:47) ou a la séquence CAACACUACUCG.

6. Oligonucléotide synthétique selon l'une quelconque des revendications 1-5, qui est modifié.

7. Oligonucléotide selon la revendication 6, dans lequel la modification comprend au moins une liaison internucléotidique choisie dans le groupe consistant en alkylphosphonate, phosphorothioate, phosphorodithioate, alkylphosphonothioate, phosphoramidate, carbamate, carbonate, phosphate triester, acétamidate, carboxyméthyl ester, et des combinaisons de ceux-ci.

8. Oligonucléotide selon la revendication 7, comprenant au moins une liaison internucléotidique phosphorothioate.

9. Oligonucléotide selon la revendication 7, dans lequel les liaisons internucléotidiques dans l'oligonucléotide sont des liaisons internucléotidiques phosphorothioate.

10. Oligonucléotide selon la revendication 6, qui comprend au moins un désoxyribonucléotide.

11. Oligonucléotide selon la revendication 6, qui comprend au moins un ribonucléotide.

12. Oligonucléotide selon la revendication 10, qui comprend en outre au moins un ribonucléotide.

13. Oligonucléotide selon la revendication 12, dans lequel une région oligodésoxyribonucléotidique est interposée entre deux régions oligoribonucléotidiques, ou sa configuration inversée.

14. Oligonuclétide selon l'une quelconque des revendications 11-13, dans lequel le ribonucléotide est un 2'-O-méthyl ribonucléotide.

15. Oligonucléotide selon la revendication 12, qui comprend au moins un 2'-O-méthyl ribonucléotide sur l'extrémité 3' de l'oligonucléotide.

16. Oligonucléotide selon la revendication 15, qui comprend en outre au moins un 2'-O-méthyl ribonucléotide sur l'extrémité 5' de l'oligonucléotide.

17. Oligonucléotide selon la revendication 1 ou 2, comprenant une séquence choisie dans le groupe consistant en SEQ ID NOS: 38, 39, 40, 41, 42, 43, 44, 45, 46, 48, 49, 50, 51, 52, 53, 54, 55, 56, 57, 58, 59, 60, 61, 62, 63, 64, 65,66 et 67, telles qu'indiquées dans le tableau 2.

18. Oligonucléotide selon la revendication 1 ou 2, comprenant une séquence choisie dans le groupe consistant en SEQ ID NOS: 134, 135, 136, 137, 138, 139, 140, 141, 142, 143, 144, 145, 146 et 147, telles qu'indiquées dans le tableau 1C.

19. Oligonucléotide selon la revendication 1 ou 2, comprenant une séquence choisie dans le groupe consistant en SEQ ID NOS: 148, 149, 150, 151, 152, 153, 154, 155, 156, 157 et 158, telles qu'indiquées dans le tableau 1D.

20. Oligonucléotide selon la revendication 6, lequel oligonucléotide est auto-stabilisé par une boucle.

21. Oligonucléotide selon la revendication 6, dans lequel la modification est choisie dans le groupe consistant en une haltère coupée, une haltère fermée, des chapeaux 2', 3' et/ou 5', des additions à la molécule sur la liaison phosphate internucléotidique, une oxydation, une oxydation/réduction, et une oxydation/amination réductrice, y compris leurs combinaisons.

22. Oligonucléotide selon la revendication 6, dans lequel au moins un nucléoside est substitué par l'inosine ou dans lequel au moins une désoxycytosine est substituée par la 5-méthyl désoxycytosine.

23. Oligonucléotide selon la revendication 6, dans lequel l'oligonucléotide est modifié par incorporation d'au moins un brin formant un triplex additionnel.

24. Oligonucléotide selon la revendication 23, ayant une séquence nucléotidique choisie dans le groupe consistant en SEQ ID NOS: 166, 167, 168, 169 et 170, telles qu'indiquées dans le tableau 1E.

25. Composition pharmaceutique comprenant au moins un oligonucléotide selon l'une quelconque des revendications 1-24 et un support pharmaceutiquement acceptable.

26. Composition pharmaceutique comprenant au moins deux oligonucléotides différents selon l'une quelconque des revendications 1-24.

27. Oligonucléotide selon l'une quelconque des revendications 1-24 pour utilisation comme composé thérapeutiquement actif, en particulier pour utilisation dans la maîtrise ou la prévention de l'infection par le virus de l'hépatite C ou sa réplication.

28. Utilisation d'un oligonucléotide selon l'une quelconque des revendications 1-24 pour la préparation d'une composition pharmaceutique pour l'inhibition de la réplication du virus de l'hépatite C dans une cellule ou pour le traitement de l'infection par le virus de l'hépatite C.

29. Procédé pour la détection de la présence du VHC dans un échantillon, comprenant les étapes de:
(a) mise en contact de l'échantillon avec un oligonucléotide synthétique selon la revendication 1, et
(b) détection de l'hybridation de l'oligonucléotide à l'acide nucléique.

30. Kit pour la détection de VHC dans un échantillon, comprenant:
(a) un oligonucléotide synthétique selon la revendication 1, et
(b) des moyens pour la détection de l'oligonucléotide hybridé avec l'acide nucléique.


### SEQUENCE LISTING

(1) GENERAL INFORMATION:
   (i) APPLICANT:
      (A) NAME: F.HOFFMANN-LA ROCHE AG
      (B) STREET: Grenzacherstrasse 124
      (C) CITY: Basle
      (D) STATE: BS
      (E) COUNTRY: Switzerland
      (F) POSTAL CODE (ZIP): CH-4070
      (G) TELEPHONE: 061 - 688 39 43
      (H) TELEFAX: 061 - 688 13 95
      (I) TELEX: 962292/965542 hlr ch

      (A) NAME: Hybridon, Inc
      (B) STREET: One Innovation Drive
      (C) CITY: Worcester
      (D) STATE: MA
      (E) COUNTRY: USA
      (F) POSTAL CODE (ZIP): 01605
      (G) TELEPHONE: 508/752-7000
      (H) TELEFAX:508/752-7001
   (ii) TITLE OF INVENTION: OLIGONUCLEOTIDES SPECIFIC FOR HEPATITIS C VIRUS
   (iii) NUMBER OF SEQUENCES: 77
   (iv) CORRESPONDENCE ADDRESS:
      (A) ADDRESSEE: F.HOFFMANN-LA ROCHE AG
      (B) STREET: Grenzacherstrasse 124
      (C) CITY: Basle
      (D) STATE: BS
      (E) COUNTRY: Switzerland
      (F) ZIP: CH-4070
   (v) COMPUTER READABLE FORM:
      (A) MEDIUM TYPE: Floppy disk
      (B) COMPUTER: Apple Macintosh
      (C) OPERATING SYSTEM: System 7.1 (Macintosh)
      (D) SOFTWARE: Word 5.1
   (vi) PRIOR APPLICATION DATA:
      (A) APPLICATION NUMBER: US 08/471,968
      (B) FILING DATE: 06.06.1995
(2) INFORMATION FOR SEQ ID NO:1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:1:
   GGTGCACGGT CTACGAGACC   20
(2) INFORMATION FOR SEQ ID NO:2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:2:
   CATGGTGCAC GGTCTACGAG 20
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:3:
   GCTCATGGTG CACGGTCTAC 20
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:4:
   GTGCTCATGG TGCACGGTCT 20
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:5:
   CGTGCTCATG GTGCACGGTC 20
(2) INFORMATION FOR SEQ ID NO:6:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:6:
   TTCGTGCTCA TGGTGCACGG 20
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:7:
   GGATTCGTGC TCATGGTGCA 20
(2) INFORMATION FOR SEQ ID NO:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:8:
   TTAGGATTCG TGCTCATGGT 20
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:9:
   GGTTTAGGAT TCGTGCTCAT 20
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:10:
   TGAGGTTTAG GATTCGTGCT 20
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:11:
   CTTTGAGGTT TAGGATTCGT 20
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:12:
   TTCTTTGAGG TTTAGGATTC 20
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:13:
   TACGTTTGGT TTTTCTTTGA 20
(2) INFORMATION FOR SEQ ID NO:14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:14:
   GTTGGTGTTA CGTTTGGTTT 20
(2) INFORMATION FOR SEQ ID NO:15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:15:
   GCACGACACT CATACTAACG 20
(2) INFORMATION FOR SEQ ID NO:16:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:16:
   GGCTGCACGA CACTCATACT 20
(2) INFORMATION FOR SEQ ID NO:17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:17:
   TGGAGGCTGC ACGACACTCA 20
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:18:
   GTCCTGGAGG CTGCACGACA 20
(2) INFORMATION FOR SEQ ID NO:19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:19:
   GGGGGTCCTG GAGGCTGCAC 20
(2) INFORMATION FOR SEQ ID NO:20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:20:
   GAGGGGGGGT CCTGGAGGCT 20
(2) INFORMATION FOR SEQ ID NO:21:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:21:
   CCGGGAGGGG GGGTCCTGGA 20
(2) INFORMATION FOR SEQ ID NO:22:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:22:
   GGCTCTCCCG GGAGGGGGGG 20
(2) INFORMATION FOR SEQ ID NO:23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:23:
   CCACTATGGC TCTCCCGGGA 20
(2) INFORMATION FOR SEQ ID NO:24:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:24:
   AACACTACTC GGCTAGCAGT 20
(2) INFORMATION FOR SEQ ID NO:25:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:25:
   ACCCAACACT ACTCGGCTAG 20
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:26:
   CGACCCAACA CTACTCGGCT 20
(2) INFORMATION FOR SEQ ID NO:27:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:27:
   CGCGACCCAA CACTACTCGG 20
(2) INFORMATION FOR SEQ ID NO:28:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:28:
   TTCGCGACCC AACACTACTC 20
(2) INFORMATION FOR SEQ ID NO:29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:29:
   CTTTCGCGAC CCAACACTAC 20
(2) INFORMATION FOR SEQ ID NO:30:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:30:
   GCCTTTCGCG ACCCAACACT 20
(2) INFORMATION FOR SEQ ID NO:31:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:31:
   AGGCCTTTCG CGACCCAACA 20
(2) INFORMATION FOR SEQ ID NO:32:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:32:
   CAAGGCCTTT CGCGACCCAA 20
(2) INFORMATION FOR SEQ ID NO:33:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:33:
   CACAAGGCCT TTCGCGACCC 20
(2) INFORMATION FOR SEQ ID NO:34
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:34:
   ACCACAAGGC CTTTCGCGAC 20
(2) INFORMATION FOR SEQ ID NO:35:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:35:
   AGTACCACAA GGCCTTTCGC 20
(2) INFORMATION FOR SEQ ID NO:36:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:36:
   GTCTACGAGA CCTCCCGGG 19
(2) INFORMATION FOR SEQ ID NO:37:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:37:
   GCACGGTCTA CGAGACCTCC 20
(2) INFORMATION FOR SEQ ID NO:38:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:38:
   GGGGUCCUGG AGNNNNNN 18
(2) INFORMATION FOR SEQ ID NO:39:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:39:
   GGGGUCCUGG AGGACCGG 18
(2) INFORMATION FOR SEQ ID NO:40:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:40:
   GACCGGGGGG UCCUGGAG 18
(2) INFORMATION FOR SEQ ID NO:41:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:41:
   GGGGUCCUGG AGAGGATT 18
(2) INFORMATION FOR SEQ ID NO:42:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:42:
   AGGATTGGGG UCCUGGAG 18
(2) INFORMATION FOR SEQ ID NO:43:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:43:
   GGGGUCCUGG AGCATGGT 18
(2) INFORMATION FOR SEQ ID NO:44:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:44:
   CATGGTGGGG UCCUGGAG 18
(2) INFORMATION FOR SEQ ID NO:45:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:45:
   GGGGUCCUGG AGCGTGCT 18
(2) INFORMATION FOR SEQ ID NO:46:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:46:
   CGTGCTGGGG UCCUGGAG 18
(2) INFORMATION FOR SEQ ID NO:47:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: RNA
   (iii) HYPOTHETICAL: NO
   (iv) ANTI-SENSE: YES
   (xi) SEQUENCE DESCRIPTION: SEQ ID NO:47:
   GGGGUCCUGG AG 12
(2) INFORMATION FOR SEQ ID NO:48:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:48:
   GGGGTCCTGG AGCATGGTGC ACGG 24
(2) INFORMATION FOR SEQ ID NO:49:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:49:
   GGGGUCCUGG AGGGTGCA 18
(2) INFORMATION FOR SEQ ID NO:50:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:50:
   GGTGCAGGGG UCCUGGAG 18
(2) INFORMATION FOR SEQ ID NO:51:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:51:
   GGGGUCCUGG AGGCTCAT 18
(2) INFORMATION FOR SEQ ID NO:52:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:52:
   GCTCATGGGG TCCTGGAG 18
(2) INFORMATION FOR SEQ ID NO:53:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:53:
   GGGGUCCUGG AGATTCGT 18
(2) INFORMATION FOR SEQ ID NO:54:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:54:
   ATTCGTGGGG UCCUGGAG 18
(2) INFORMATION FOR SEQ ID NO:55:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:55:
   GGGGTCCTGG AGAGGATTCG TGCT 24
(2) INFORMATION FOR SEQ ID NO:56:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:56:
   GGGGUCCUGG AGCGTGCTCA TGGT 24
(2) INFORMATION FOR SEQ ID NO:57:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:57:
   CATGGTGCAC GGGGGGTCCT GGAG 24
(2) INFORMATION FOR SEQ ID NO:58:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:58:
   TGGATTCGTG CAGGGGTCCT GGAG 24
(2) INFORMATION FOR SEQ ID NO:59:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:59:
   CGTGCTCATG GTGGGGTCCT GGAG 24
(2) INFORMATION FOR SEQ ID NO:60:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:60:
   GGGGTCCTGG AGATTCGTGC TCAT 24
(2) INFORMATION FOR SEQ ID NO:61:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:61:
   ATTCGTGCTC ATGGGGTCCT GGAG 24
(2) INFORMATION FOR SEQ ID NO:62:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:62:
   GGGGTCCTGG AGTGGTGCAC GGTC 24
(2) INFORMATION FOR SEQ ID NO:63:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:63:
   TGGTGCACGG TCGGGGTCCT GGAG 24
(2) INFORMATION FOR SEQ ID NO:64:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:64:
   GGGGUCCUGG AGGCTCATGG TGCA 24
(2) INFORMATION FOR SEQ ID NO:65:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:65:
   GCTCATGGTG CAGGGGUCCU GGAG 24
(2) INFORMATION FOR SEQ ID NO:66:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:66:
   GGGGTCCTGG AGGCACGGTC TACG 24
(2) INFORMATION FOR SEQ ID NO:67:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:67:
   GGGGUCCUGG AGNNNNNNNN NNNN 24
(2) INFORMATION FOR SEQ ID NO:68:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:68:
   TTCGCGACCC AACACTACTC GGCTAGCA 28
(2) INFORMATION FOR SEQ ID NO:69:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:69:
   CATGGCTAGA CGCTTTCTGC 20
(2) INFORMATION FOR SEQ ID NO:70:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:70:
   CTCGCGGGGG CACGCCCAAA 20
(2) INFORMATION FOR SEQ ID NO:71:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:71:
   TGAGCGGGTT GATCCAAGAA 20
(2) INFORMATION FOR SEQ ID NO:72:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:72:
   GATCCAAGAA AGGACCCGGT 20
(2) INFORMATION FOR SEQ ID NO:73:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:73:
   GGCTAGCAGT CTCGCGGGGG 20
(2) INFORMATION FOR SEQ ID NO:74:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:74:
   GCCTTTCGCG ACCCAACACT ACTCGGCT 28
(2) INFORMATION FOR SEQ ID NO:75:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:75:
   CTTTCGCGAC CCAACACTAC TCGG 24
(2) INFORMATION FOR SEQ ID NO:76:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:76:
   CGCGACCCAA CACTAC 16
(2) INFORMATION FOR SEQ ID NO:77:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:77:
   GGGGCACTCG CAAGCACCCT 20